# EUROPEAN PATENT SPECIFICATION

(11) **EP 2 950 777 B1**
(45) Date of publication and mention of the grant of the patent: **23.06.2021**
(21) Application number: 14701730.5
(22) Date of filing: 24.01.2014
(51) Int. Cl.: A61P 1/02, A61K 8/43, A61Q 11/00, A61K 9/00, A61K 31/155, A61K 33/20

(54) **PERIODONTAL DISEASE TREATMENT**
ZAHNFLEISCHERKRANKUNGSBEHANDLUNG
TRAITEMENT DE MALADIE PARODONTALE

(30) Priority: 30.01.2013 EP 13153249
(43) Date of publication of application: 09.12.2015
(73) Proprietor: Straumann Holding AG, 4002 Basel (CH)
(72) Inventor: BERG, Marianne, CH-4002 Basel (CH); RAEBER, George, CH-4002 Basel (CH)
(74) Representative: Aera A/S
(86) International application number: PCT/EP2014/051421
(87) International publication number: WO 2014/118090

(56) References cited:
- WO-A1-2008/003688
- WO-A1-2008/096022
- WO-A2-03/061506
- DE-A1-102006 029 344
- US-A1- 2011 020 474
- None

## Description

### Technical field

The present invention relates to the field of treating periodontal diseases, such as periimplant infections, periimplantitis, gingivitis, periodontitis or periimplant mucositis, caused by microorganisms which colonize the tooth and/or implant surface at, above and/or below the gingival margin, and which reside in a biofilm, either subgingival and/or supragingival.

It is to be understood that the method and kit of parts presented herein can of course also be used for sustainably removing and/or destroying, killing and/or disrupting a pathogenic biofilm at a site of microbial infection in a patient with an orthopaedic implant.

### Background

Periodontal diseases are infections caused by microorganisms that colonize the tooth or implant surface at or below the gingival margin. While these infections have many properties in common with other infectious diseases, they exhibit unique properties conferred by their site of colonization and the nature of the environment in which they reside. The onset of the diseases is usually delayed for prolonged periods of time after initial colonization by the pathogens(s).

The major characteristics of these diseases are that they are caused by organisms that reside in biofilms either subgingival and/or supragingival. Their treatment is complex in that physical, antimicrobial and ecological approaches are required. The biofilm provides protection to colonizing species from competing microorganisms and from environmental factors such as host defense mechanisms, and from potential toxic substances, such as chemicals or antibiotics. They are composed of microcolonies of bacterial cells of one or more species that are distributed in a shape matrix or glycocalyx.

The associations of bacteria within mixed dental biofilms are complex. According to Socransky and Haffajee, 2000, and 2000, six closely associated groups of bacterial species have been recognized. These include *Actinomyces, Streptococci, Capnocytophaga, Actinobacillus actinomycetemcomitans* serotype a, *Eikenella corrodens* and *Campylobacter concisus, Veillonella parvula* and *Actinomyces odentolycticus.* These groups are dominated by gram positive bacteria and recognized as early colonizers on the tooth and/or implant surface and certain complexes are observed together more frequently than others in the subgingival plaque. Similar relationships can be demonstrated in *in vitro* experiments with oral bacterial species. The early colonizers attach to a clean surface and provide a conditioning film to which other species can then bind. With time, oral biofilms become more complex and are joined by or replaced by other species. Thus at a later stage in dental biofilm development, gram-negative species become numerically dominant.

Biofilm initiated diseases are by no means unique to the oral cavity. Approx. 65% of infections that affect the human are caused by organisms growing in biofilms. These include dental caries, periodontal disease, otitis media, musculoskeletal infections, necrotizing fasciitis, biliary tract infection, osteomyelitis, bacterial prostatitis, native valve endocarditis, meloidosis, prosthetic as well as orthopaedic complications and cystic fibrosis pneumonia. Characteristics are persistence and chronicity of the infections as well as the difficulty in eradication.

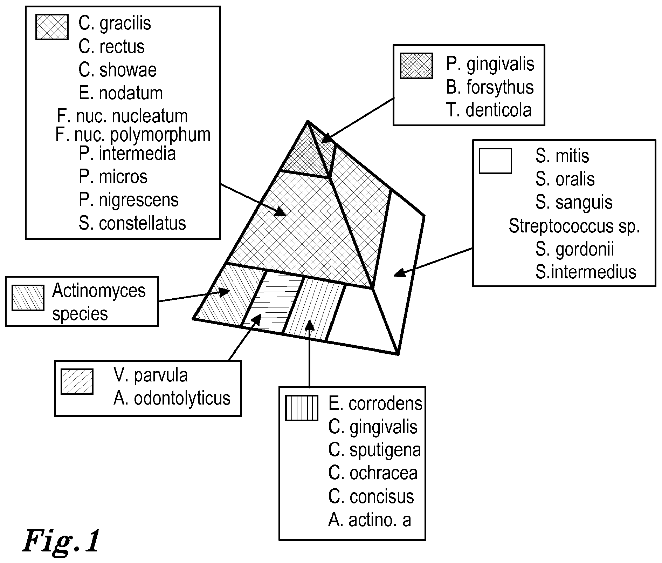

Dental plaque, a sticky colorless film is caused by bacterial deposits accumulating on tooth or implant surfaces along the gingival margins and results in the destruction of tooth-supporting tissues. Dental plaque formation starts in cracks, grooves and surface roughness on teeth and/or dental implants. In any given plaque sample, it is not uncommon to detect 30 or more bacterial species. Thus, the biofilm that colonizes the tooth surface may be among the most complex biofilms that exist in nature. The bacteria associated with periodontal diseases reside in biofilms both above and below the gingival margin. The supragingival biofilm is attached to the tooth or the implant and predominated by *Actinomyces species,* the subgingival biofilm is typically more complex and can either attach to the tooth or implant, or to the gingival tissue. Although it is today not given, which of the colonizing microbe species cause the periodontal diseases; three species are singled out by studies to be strongly associated with disease status progression and unsuccessful therapies: *A. actinomycetemcomitans, P. gingivalis* and *B*. *forsythus.* Also, *F. nucleatum, Campylobacter rectus, P. Intermedia, P. nigrescens, Eubacterium nodatum, P. micros* and various spirochetes have been singled out so far. For a schematic summary, see also fig.1.

The destruction of tooth-supporting tissues results in a deepening of the space (periodontal pocket) between the root of the tooth and the gum tissue. Second to tooth decay, periodontal diseases are the most frequent oral diseases and may lead to partial or complete tooth or bone loss. It has been estimated that they affect as much as between 70-90% of the world population, and they are the major cause of tooth loss in people over 35 years of age. The most common forms of periodontal diseases are gingivitis and periodontitis.

Gingivitis is the mildest form of periodontal disease, causing the gingiva to become red, swollen, and bleed easily. Gingivitis, if untreated, may develop into periodontitis. In periodontitis the infection has progressed to involve the oral tissues which retain the teeth in the jawbone. If untreated, periodontitis ultimately leads to loss of the affected tooth.

Chronic periodontitis, the most frequently occurring form of periodontitis, results in inflammation within the supporting tissues of the teeth, progressive loss of attachment as well as progressive alveolar bone resorption. This form of periodontitis is characterized by pocket formation and/or recession of the gingiva. As the destruction advances, the mobility and movement of teeth increase, finally causing spontaneous loss of a tooth or a necessity of tooth extraction.

Treatment of periodontal diseases usually involves the removal of bacterial deposits and dental calculus. However, it is difficult to have full access for treating deeper periodontal pockets, resulting in remaining bacteria that may re-infect the tissue. This is of course also the case for other bacterially infected tissues, where an incomplete removal of bacteria or dead or damaged tissue may cause problems for healing and give rise to re-infection. Therefore, this treatment is often combined with surgical procedures to open the periodontal pocket. The area is then scraped or otherwise mechanically freed from bacterial deposits and calculus but also granulation tissue and bacterial toxin.

Patients with dental implants are susceptible to developing conditions similar to the above described periodontal diseases but which instead attack the tissues surrounding the implant. One such disease is periimplant mucositis. This condition involves the presence of inflammation in the mucosa at an implant but with no signs of loss of supporting bone.

Patients with implants can also suffer from a condition called peri-implantitis, which is caused by the colonization of bacteria of the implant's surface. Inflammation in the bone surrounding the implant then causes loss of bone which ultimately may lead to failure of the implant. Peri-implantitis can stem from an existing periodontitis infection or patients can develop peri-implantitis without a previous history of periodontitis.

The surface of dental implants, or the vicinity thereof, has thus sometimes to be cleaned after placing, for example, when an infection or contamination occurs, causing peri-implantitis. In these cases, the surface of the ailing implant has to be cleaned of microbes and other contaminants to stop the progression of the disease. Failure to clean the implant surface will eventually lead to loss of bone and implant, and make further alternative treatments difficult and sometimes even impossible.

Traditionally, dentists and surgeons utilize the same cleaning tools that are used in relation to treatment of periodontitis, for example a curette or a spatula, but of lately, other more adapted cleaning and debridement tools have been introduced in the field, such as titanium-bristled brushes. Other implant cleaning methods include use of abrasive blasting, chemical cleaning agents and lasers, as well as ultrasonic devises. Each of these methods has drawbacks; for example, although a laser can be used to kill bacteria, this method in isolation does not necessarily remove the bacteria, and thus a biofilm can remain on the implant which can hinder osseointegration and may act as a source of later infection. Ideally, a cleaning and/or decontamination agent specifically targets bacteria without damaging the tissue. The selection of agent as well as of method is typically based on a risk/benefit evaluation by the skilled artisan. This limits the number of suitable cleaning agents available, and it can be a difficult and time consuming task to identify suitable agents and their suitable application parameters. Further, there is some doubt that the use of chemical cleaning agents in isolation would be able to diffuse through a thick biofilm and thus enable its removal from the implant. Known abrasive blasting methods include the use of glycine particles in pressurized air. Such methods however carry a risk of emphysema, namely an abnormal distension of the surrounding tissues with gas.

Another currently employed treatment is the systemic or local treatment with antibiotics. Nonetheless, antimicrobial agents are unlikely to be effective at normal dosage, as the minimum inhibitory concentration for antibiotics for an organism in biofilm mode might be 1000-1500 times higher than for the same organism in the planktonic state. What is more, although antibiotics have been reported to be useful in treating periodontal infections, their use has the potential to lead to the resistance of not only one but several species incorporated in the biofilm, due to the improved ability of the biofilm microbes to effectively pass on genetic advantages to each other (for a review, see e.g. Socransky S.S.and Haffajee A.D., 2000).

WO 2008/096022 A1 discloses an endodontic irrigation treatment comprising sequential rinsing with a natrium hypochlorite preparation and a chlorhexidine preparation, solutions of sodium hypochlorite are employed at concentrations of 2% and 5%.

WO 03/061506 A2 discloses anti-infective and antibiofilm irrigation preparation(s) for tooth and bone surfaces prepared for endodontic treatment, root canal therapy, periodontic procedure, tooth restoration or reconstruction, comprising a disinfectant, a detergent and an acid (ADD); a preferred antimicrobial component is chlorhexidine gluconate (0,12% solution), the treatment alternates NaClO and an antibacterial/antimicrobial solution.

### Summary of invention

The present invention for the first time discloses a method and a kit of parts for use in treating a periodontal disease, including periimplant infections, such as periimplantitis, gingivitis, periodontitis and/or periimplant mucositis, caused by microorganisms which colonize the tooth and/or implant surface at, above and/or below the gingival margin, and which reside in a biofilm, either subgingival and/or supragingival. Said treatment is characterized by employing an at least partially surgical process leading to substantively removing, destroying, killing and/or disrupting a pathogenic biofilm at a site of microbial infection in a patient suffering from a periodontal disease and inhibiting, obstructing, impeding and/or preventing the growth and/or regrowth of a pathogenic biofilm at a site of microbial infection in a patient. Said method comprises cleaning and/or debriding the site of microbial infection mechanically and sequentially using first at least one cleaning agent with an immediate bactericidal effect, such as a sodium hypochlorite (NaClO) solution, and thereafter at least one cleaning agent with a sustainable bactericidal effect, such as a chlorhexidine (CHX) solution.

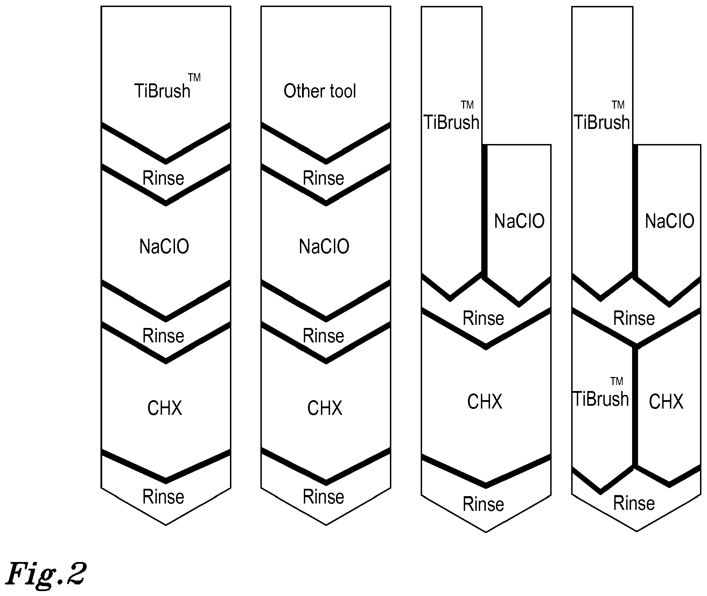

The treatment disclosed herein comprises optionally first surgically assessing, or alternatively directly cleaning and/or debriding a site of microbial infection in a patient suffering from a periodontal disease with a mechanical tool, and employing at least two cleaning and/or disinfecting agents in the following sequential order: first a NaClO solution, and thereafter a CHX solution. The cleaning and/or debridement tool to be used can e.g. be a suitable brush, such as a titanium-bristled brush, a curette, a spatula and/or an ultrasonic device. The mechanical cleaning can e.g. be performed in a pre-treatment step and/or simultaneously, or after to the employing of one or both of the at least two cleaning and/or disinfecting agents. Optionally, the treatment further comprises rinsing the site of the microbial infection before, in between, during and/or after the application of the cleaning and/or disinfecting agents (see figure 2 illustrating examples of possible methods and application steps).

The kit of parts disclosed for use herein comprises two cleaning and/or disinfecting agents for use at the site of microbial infection, said agents are selected from: a) NaClO solution, and b) CHX solution, and in one embodiment an instruction, such as a written instruction, describing the use of the two cleaning agents in a given sequential order of first applying a), and thereafter applying b), optionally rinsing the site of the microbial infection both before, in between, during and/or after the application of the cleaning and/or disinfecting agent(s). Optionally again, said kit further comprises a cleaning and/or debridement tool, such as a suitable brush, and/or a suitable rinsing solution.

The method and kit of parts for use presented herein can further be used for sustainably removing, killing, destroying and/or disrupting, as well as inhibiting, obstructing, impeding and/or preventing the growth and/or regrowth of a pathogenic biofilm at a site of a microbial infection in a patient with an orthopaedic implant.

### Definitions

"Peri-implantitis" or "periimplantitis" is a dental term used to describe the destructive inflammatory process affecting the soft and hard tissues surrounding dental implants. Compared to mucositis, the definition of peri-implantitis includes bone loss. Among others, smoking, accumulation of bacterial biofilms (plaque), oral hygiene and periodontal status are influential factors. In the present context, the term "periodontal diseases" encompasses periimplant infections, such as periimplantitis.

"Prolonged antimicrobial activity" is a phenomenon called substantivity. In the present application, this term is used interchangeably with sustainable bactericidal and/or antimicrobial effect.

In the present context, a biofilm is an extracellular matrix community of sessile, stable attached microorganisms, such as bacteria, virus or fungi, embedded in a self-produced matrix consisting of various components, including extracellular polymeric substances.

The term "cleaning agent(s)" is in the present context interchangeably used with "cleaning solution(s)" and describes substance(s), usually liquid(s), that are used to removing, destroying, killing and/or disrupting microorganisms, such as bacteria and/or biofilm, as well as cellular and other debris on surfaces.

It is to be understood that the terminology used herein is for the purpose of describing particular embodiments only and is not intended to limit the scope of the present invention which will be limited only by the appended claims. It must be noted that as used herein and in the appended claims, the singular forms "a", "an", and "the" include plural references unless the context clearly dictates otherwise.

The term "patient" as used herein refers to any mammal. Examples of mammals are humans, farm animals and domestic animals.

The abbreviation "CMF" herein stands for craniomaxillofacial complex, i.e. the anatomical area of the mouth, jaws, face, skull, as well as associated structures.

Orthopedic implants are in the present context defined as medical devices used to replace or provide fixation of bone, to support a damaged bone and/or to replace articulating surfaces of a joint. An orthopaedic implant is in the present context selected from the group consisting of arthoplasty, bone plates, bone screws and nailing.

The abbreviation "CFU" is known in the art to stand for colony-forming unit (CFU), which is an estimate of viable bacterial or fungal numbers. The results are given as CFU/mL (colony-forming units per milliliter) for liquids, and CFU/g (colony-forming units per gram) for solids to reflect this uncertainty (rather than cells/mL or cells/g).

In the present context, SLA® refers to a titanium surface which is produced by a large grit sand-blasting process with corundum particles that leads to a macro-roughness on the titanium surface. This is followed by a strong acid-etching bath with a mixture of HCl/H₂SO₄ at elevated temperature for several minutes. This produces 2-4 µm fine micro-pits superimposed on the rough-blasted surface. The surface is not micro-porous and therefore provides no enclosed volumes to reduce vulnerability to bacteria.

In the present context, Clean Implant Surface is abbreviated CIS.

### Figure legends

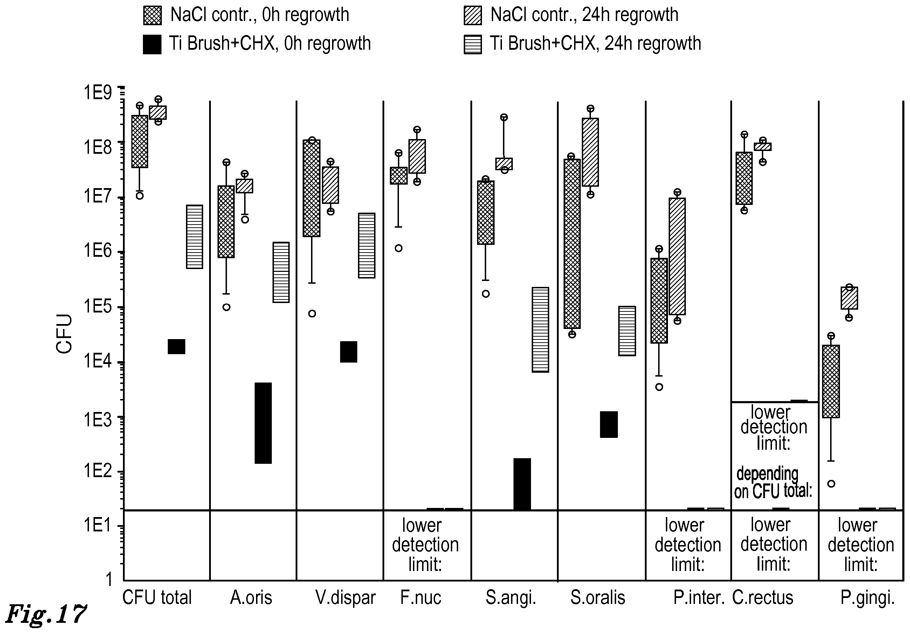

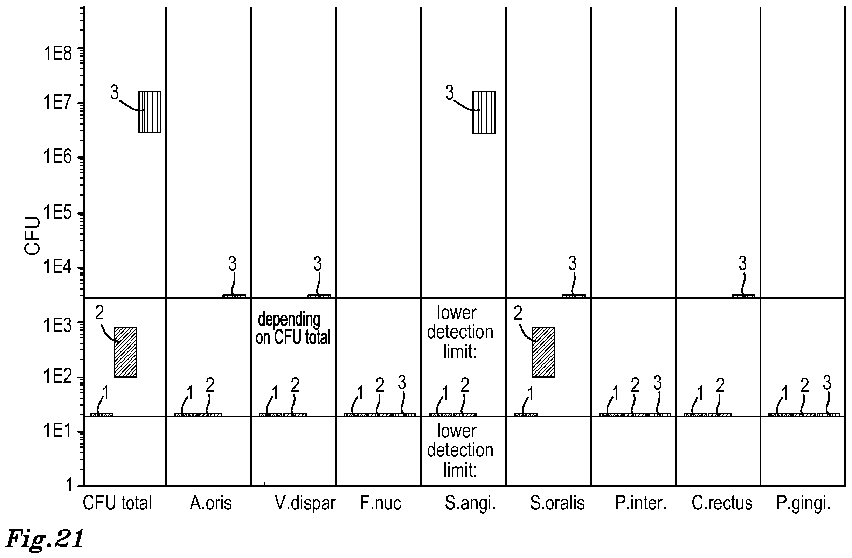

- Figure 1:: Shows the biofilm model which is found in Socransky et al., Periodontology 2000, Vol. 28, 2002, 12-55*.* Several of the bacteria categorized herein are tested in the biofilm models used in the experimental section. The tested strains are chosen as representatives for the category that they are allotted to. Bacterial strains categorized as belonging to the supragingival model are tested in experiments 1 and 2. Bacterial strains categorized as belonging to the subgingival model are tested in experiments 3-6.
- Figure 2:: A schematic visualisation of different embodiments of the method of the present application.
- Figure 3A:: Data on CFU count obtained immediately after cleaning agent treatment in three experiments: twice with the supragingival model (experiment 1 and experiment 2) and once with the subgingival model (experiment 3). Values display mean of n=3. Values below detection limit (<2.0E1) are shown as 2.0E1.
- Figure 3B:: Data on CFU count obtained 24 hours after cleaning agent treatment in three experiments: twice with the supragingival model (experiment 1 and experiment 2) and once with the subgingival model (experiment 3). Values display mean of n=3. Values below detection limit (<2.0E1) are shown as 2.0E1.
- Figure 4A:: Data on CFU count obtained immediately after TiBrush® and cleaning agent treatment in two experiments with the subgingival model (experiment 4 and experiment 5). Values display mean of n=3. Values below detection limit (<2.0E1) are shown as 2.0E1.
- Figure 4B:: Data on CFU count obtained 24 hours after TiBrush® and cleaning agent treatment in two experiments with the subgingival model (experiment 4 and experiment 5). Values display mean of n=3. Values below detection limit (<2.0E1) are shown as 2.0E1.
- Figure 4C:: Data on CFU count obtained 24 hours, 48 hours and 72 hours after TiBrush® and cleaning agent treatment in one experiment with the subgingival model (experiment 6). Values display mean of n=3. Values below detection limit (<2.0E1) are shown as 2.0E1.
- Figure 5:: Discloses analysis on individual bacteria CFU count (see experiment 6).
1) Ti Brush + NaClO (0.1%) 0h regrowth
2) Ti Brush + NaClO (0.1%) 24h regrowth
3) Ti Brush + NaClO (1%) + CHX 0h regrowth
4) Ti Brush + NaClO (1%) + CHX 24h regrowth
- Figure 6:: Data on Clean implant surface (CIS) after treatment of Straumann SLA® discs with TiBrush® and cleaning agents in a human in-vivo splint model. For more details see experiment 7.
- Figure 7:: Discloses an evaluation of biofilm decontamination with various antimicrobial agents tested in a 70/30 biofilm model CFU (log10) on Columbia Blood Agar / N=3. (Experiment 1)
1a) CHX, 1b) CHX, 24h
2a) H₂O₂, 2b) H₂O₂, 24h
3a) Na-hypo-Cl, 3b) Na-hypo-Cl, 24h
4a) phys. NaCl, 4b) phys. NaCl, 24h
- Figure 8:: Discloses an evaluation of biofilm decontamination with various antimicrobial agents tested in a 70/30 biofilm model CFU (log10) on Columbia Blood Agar / N=3. (Experiment 2)
1a) CHX, 0.2%, 1b) CHX, 0.2%, 24h
2a) CHX, 1.0%, 2b) CHX, 1.0%, 24h
3a) H₂O₂, 0.1%, 3b) H₂O₂, 0.1%, 24h
4a) H₂O₂, 1.0%, 4b) H₂O₂, 1.0%, 24h
5a) NaClO, 0.1%, 5b) NaClO, 0.1%, 24h
6a) NaClO, 1.0%, 6b) NaClO, 1.0%, 24h
7a) NaClO, 3.0%, 7b) NaClO, 3.0%, 24h
8a) phys. NaCl, 8b) phys. NaCl, 24h
- Figure 9:: Discloses treatment 1 + 2: Chlorhexidine digluconate tested in a 70/30 subgingival biofilm model CFU (log10) on Agar / N=3. (Experiment 3)
- Figure 10:: Discloses treatment 3 + 6: H₂O₂ tested in a 70/30 subgingival biofilm model CFU (log10) on Agar / N=3. (Experiment 3)
- Figure 11:: Discloses treatment 4 + 5: Sodium hypochlorite tested in a 70/30 subgingival biofilm model CFU (log10) on Agar / N=3. (Experiment 3)
1) NaOCI, 0.1%, 2) NaOCI, 0.1%, 24h
3) NaOCI, 1%, 4) NaOCI, 1%, 24h
- Figure 12:: Discloses treatment 7 + 8: phys. NaCl tested in a 70/30 subgingival biofilm model CFU (log10) on Agar / N=3. (Experiment 3)
- Figure 13:: Discloses an overview: total CFU tested in a 70/30 subgingival biofilm model CFU (log10) on Columbia Blood Agar / N=3. (Experiment 3)
The tartan bars reflect the immediate effect after a 1min exposure to the antimicrobial solution; the striped bars the sustained effect after 24 h.
- Figure 14:: NaCl 0.9% control + TiBrush® tested in a 70/30 subgingival biofilm model CFU (log10) on Agar / N=3 or 6. (Experiment 4)
- Figure 15:: TiBrush® + CHX 0.2% or NaClO 1% tested in a 70/30 subgingival biofilm model CFU (log10) on Agar / N=3. (Experiment 4)
- Figure 16:: Discloses an overview: total CFU tested in a 70/30 subgingival biofilm model CFU (log10) on Agar / N=3. (Experiment 4)
1a) NaCl contr., 0h regrowth, 1b) NaCl contr., 24h regrowth
2a) TiBrush®, 0h regrowth, 2b) TiBrush®, 24h regrowth
3a) TiBrush®+CHX, 0h regrowth, 3b) TiBrush®+CHX, 24h regrowth
4a) TiBrush®+NaClO(0.1%), 0h regrowth, 4b) TiBrush®+NaClO(0.1%), 24h regrowth
5a) TiBrush®+NaClO(1%), 0h regrowth, 5b) TiBrush®+NaClO(1%), 24h regrowth
6a) TiBrush®+NaClO(1%)+CHX, 0h regrowth,
6b) TiBrush®+NaClO(1%)+CHX 24h regrowth
- Figure 17:: NaCl 0.9% control and TiBrush® + CHX 0.2% tested in a 70/30 subgingival biofilm model CFU (log10) on Agar / N=3 (6). (Experiment 5)

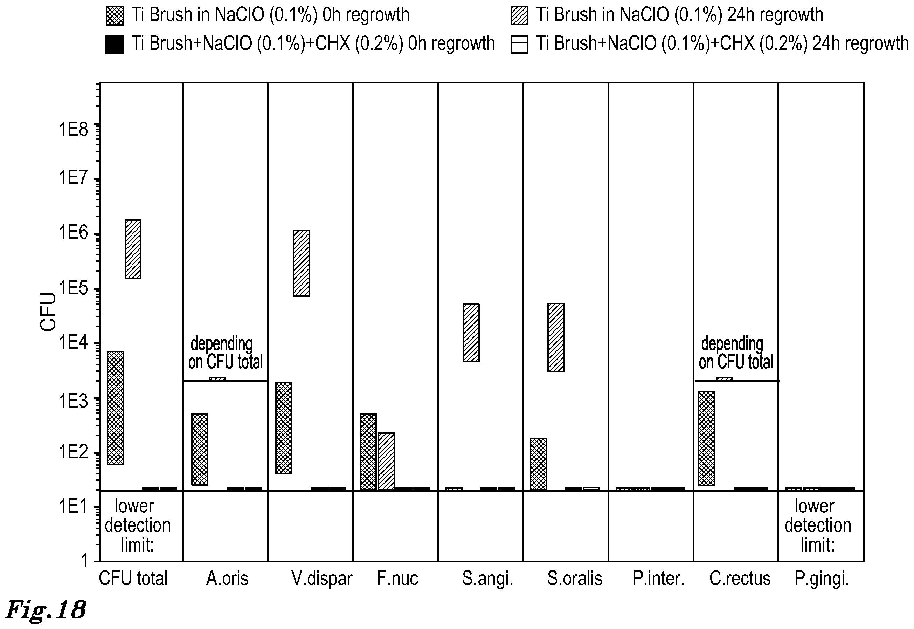

- Figure 18:: TiBrush® + NaClO 0.1% + CHX 0.2% and TiBrush® in NaClO 0.1% tested in a 70/30 subgingival biofilm model CFU (log10) on Agar / N=3. (Experiment 5)

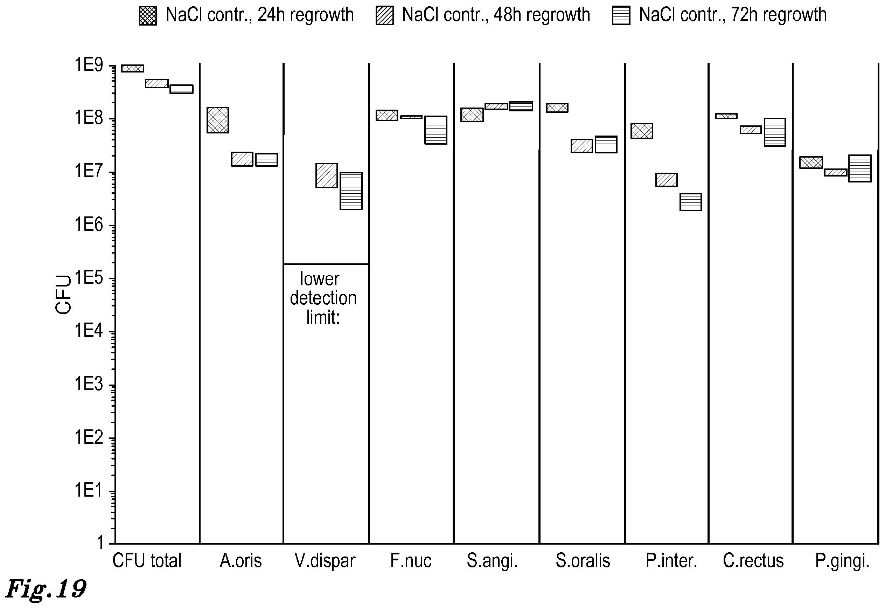

- Figure 19:: NaCl 0.9% control tested in a 70/30 subgingival biofilm model CFU (log10) on Agar / N=3. (Experiment 6)

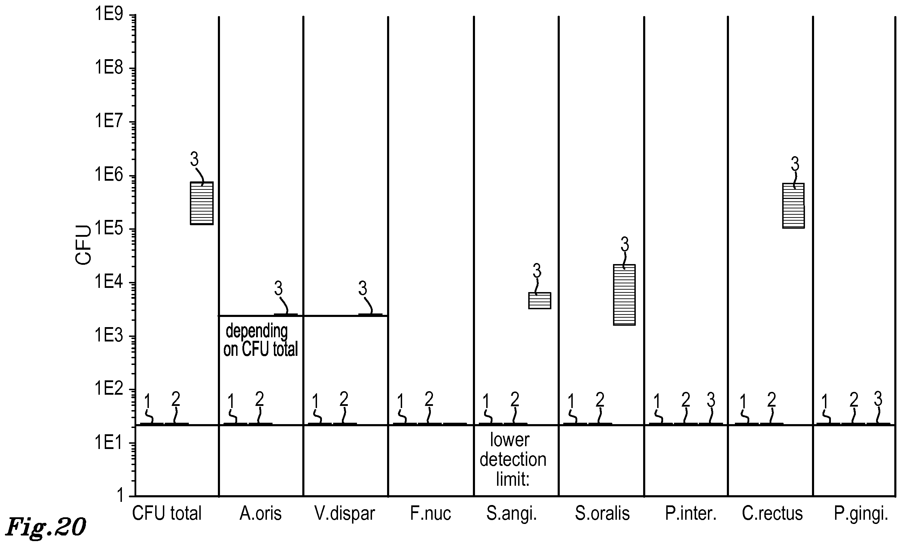

- Figure 20:: TiBrush® + NaClO 0.1% + CHX 0.2% tested in a 70/30 subgingival biofilm model CFU (log10) on Agar / N=3. (Experiment 6)
1) TiBrush®+NaClO (0.1%) +CHX (0.2%), 24h regrowth
2) TiBrush®+NaClO (0.1%) +CHX (0.2%), 48h regrowth
3) TiBrush®+NaClO (0.1%) +CHX (0.2%), 72h regrowth
- Figure 21:: Discloses treatment 3: TiBrush® + NaClO 1.0% + CHX 0.2% tested in a 70/30 subgingival biofilm model CFU (log10) on Agar / N=3. (Experiment 6)
1) TiBrush®+NaClO (1%) +CHX (0.2%), 24h regrowth
2) TiBrush®+NaClO (1%) +CHX (0.2%), 48h regrowth
3) TiBrush®+NaClO (1%) +CHX (0.2%), 72h regrowth

### Detailed description of invention

The present invention, for the first time, discloses an effective method and a kit of parts for use in treating a periodontal disease, such as periimplantitis, gingivitis, periodontitis and/or periimplant mucositis, caused by microorganisms, which colonize the tooth and/or implant surface at, above and/or below the gingival margin, and which reside in a biofilm either subgingival and/or supragingival. The method and the intended use of the kit of parts are characterized by sustainably destroying, killing, removing and/or disrupting a pathogenic biofilm at a site of microbial infection in a patient suffering from a periodontal disease. Also, the method and the intended use of the kit of parts is characterized by effectively and sustainably inhibiting, obstructing, impeding and/or preventing the growth and/or regrowth of a pathogenic biofilm at the same site of microbial infection in a patient suffering from a periodontal disease.

The inventors have surprisingly found that a pathogenic biofilm at a site of microbial infection in a patient suffering from a periodontal disease can substantively be removed, killed and/or destroyed and its potential growth and/or regrowth effectively prevented, inhibited, obstructed, impeded and/or reduced by employing a method comprising several steps, individually at least partially known to be beneficial for treating different oral and/or dental diseases, but together achieving a novel, synergistic and lasting bactericidal and/or bacteriostatic effect on a pathogenic biofilm at a site of microbial infection in a patient.

The presently disclosed method typically comprises surgically assessing a site of microbial infection and cleaning and/or debriding said site mechanically, as well as sequentially using one cleaning and/or disinfecting agent with an immediate bactericidal effect, such as a NaClO solution, and thereafter using at least one cleaning and/or disinfecting agent with a sustainable bactericidal effect, such as a CHX solution.

The method presented herein for the first time describes an effective and substantive local treatment for periodontal diseases, in particular periimplantitis. On the other hand, should the need arise, it is also possible to combine the present method with a systemic and/or local administration of antibiotics and/or antimicrobials. It is envisioned that such a combination would support a higher efficiency of the administered dose of antibiotics and/or antimicrobials, thus optionally facilitating a lowering of the dose administered to the patient in need thereof.

The present invention, for the first time, discloses that a sequential use of first at least one cleaning and/or disinfecting agent with an immediate bactericidal effect, such as a NaClO solution, and thereafter using at least one cleaning and/or disinfecting agent with a sustainable bactericidal effect, such as a CHX solution, leads to a sustainable removal, disruption, killing and/or inhibition of growth and/or regrowth of a pathogenic biofilm at the site of a microbial infection in the oral cavity and/or in the craniomaxillofacial (CMF) area.

As is clearly documented in the experimental section (experiments 1-6), the present inventors, for the first time, were able to demonstrate in an established biofilm model (see e.g. Guggenheim et al, 2004, 2001a, 2009; and Shapiro et al., 2002), and in a human splint model (experiment 7, Schwartz et al., 2006), that the sequential use of first cleaning and/or debriding the implant surface mechanically, and then applying at least one cleaning and/or disinfecting agent with an immediate bactericidal effect, such as NaClO solution, and thereafter at least one cleaning and/or disinfecting agent with a sustainable bactericidal effect, such as CHX solution, leads to a sustainable destruction, removal, killing and/or inhibition of growth and/or regrowth of a pathogenic biofilm.

The present findings disclose the selective effects of this method of treatment and/or kit of parts on single species (see e.g. figures 19-21). E.g. putative pathogenic, Gram-negative anaerobic bacteria, which are in the field known to be strongly associated with inflammation and peri-implantitis (*F. nucleatum, P. intermedia. and P. gingivalis*), were after a 72 h regrowth period still below detection level. *Campylobacter rectus,* a micro-aerophilic species, was completely suppressed after 48 h and was significantly reduced in the biofilms after 72 h. The Gram-positive species, as well as *V. dispar,* were able to regrow only after 72 h in a treatment dependent manner, but these species are only weakly associated with inflammation or even regarded as part of a microbiota associated with health.

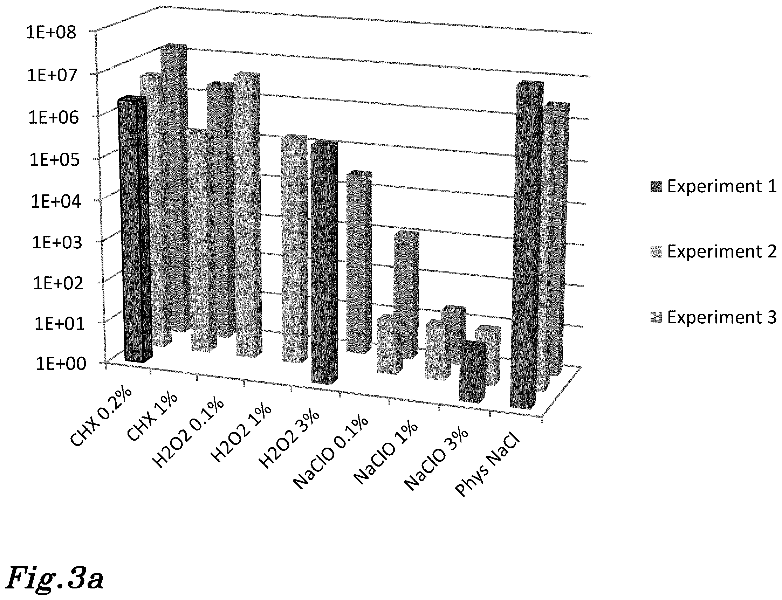

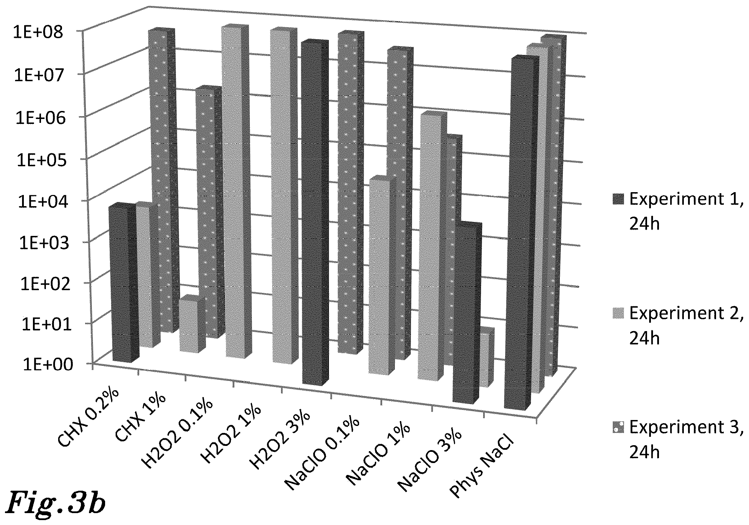

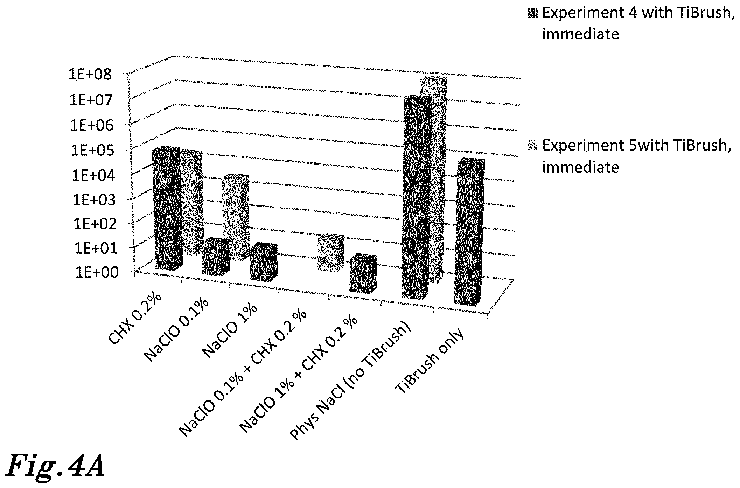

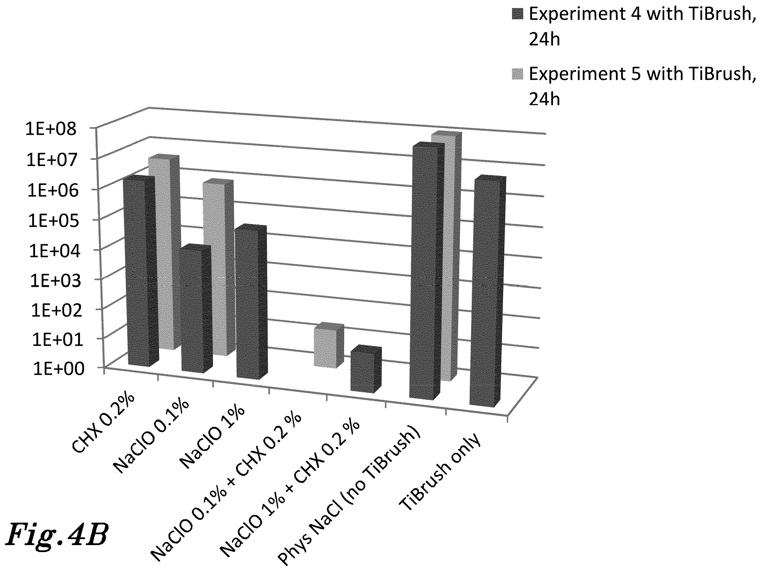

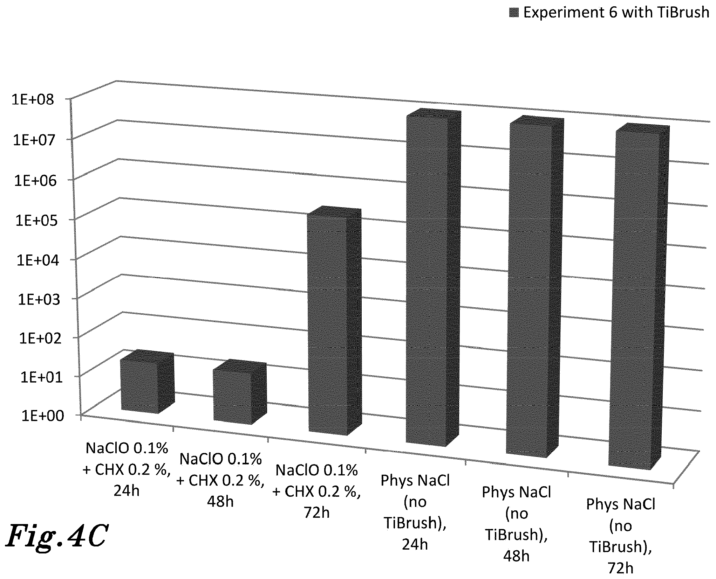

As is illustrated in summary in figures 3 and 4, experiments on engineered biofilms suggest that a combination of an immediate and a sustained acting cleaning agent is synergistically effective.

A comparison of CFU data after treatment with different concentrations of CHX, H₂O₂ and NaClO, as well as physiological NaCl as control, is shown in figures 3a and 3b. An immediate effect of a cleaning agent in a supragingival model is illustrated in figure 3a: Herein, it is demonstrated that NaClO solution reduces CFU counts the most, with higher concentrations also effective on subgingival strains. CHX solution and H₂O₂ show a similar effect on CFU immediately after treatment. In contrast, a sustainable effect of the tested cleaning agent in a supragingival model is illustrated in figure 3b: Herein it is shown that despite the limited immediate influence on CFU counts for CHX solution, a sustained effect is seen for 0.2% and 1% in the supragingival model. Interestingly, this is not observed in the subgingival model. In the treatment with NaClO solution 0.1% and 1%, a biofilm re-establishes even though the CFU count was very low after immediate treatment of the supragingival film. All other tested agents led to a biofilm similar to the control after 24 hours. CHX solution and control show similar biofilm immediately after cleaning, however, a clear difference is observed in bacterial re-growth after 24h. Thus, albeit NaClO solution seems to remove bacteria, it seems to have a very weak sustained effect.

In summary, after treatment with NaClO, a strong immediate CFU reduction is seen in the supragingival as well as subgingival biofilm model. However, at concentration of 0.1% NaClO and 1% NaClO, a bacterial regrowth is seen after 24 hours. NaClO seems to remove bacteria, but have no sustained effect on its own. After treatment with 0.2% CHX and 1% CHX, no significant immediate CFU influence was seen. However after 24 hours, a clear CFU reduction was observed in the supragingival biofilm model
Figures 4a and 4b disclose additional experiments on working mechanism for a combination of NaClO and CHX, showing that the sequential usage leads to no CFU counts after 24 h on SLA® surface in a subgingival model. A comparison is shown between CFU data after treatment with TiBrush® followed by different concentrations of CHX, H₂O₂ and NaClO as well as physiological NaCl as control.

In figure 4a, an immediate effect of the tested cleaning agents is illustrated: Furthermore, it can be seen that adding the mechanical treatment step of TiBrush®, has a positive effect, generally lowering the CFU counts. Immediate and 24 hours data with TiBrush® + 0.1% NaClO + 0.2% CHX as well as with TiBrush® + 1% NaClO + 0.2% CHX show that sequential treatment of subgingival biofilm with TiBrush® + 1% NaClO + 0.2% CHX shows a strong immediate, as well as a prolonged bactericidal effect after 24 hours.

The sustainable effect of the tested cleaning agents is shown in figure 4b as well as in figure 4c: This sustainable effect was not seen using the combination of TiBrush® + 1% NaClO alone, or using TiBrush® + 0.2% CHX alone, having an increased bacterial count after 24h. However, performing a sequential treatment with TiBrush®, NaClO solution 1% and CHX solution 0.2%, results in no increase in CFU count, i.e. the situation at immediate cleaning is maintained for 24h. For all other tested agents, a regrowth of biofilm was seen within 24h, also after combined mechanical and chemical treatment (results not shown). Thus, only a combination of NaClO and CHX is proven to be effective and sustainable over time.

By lowering the concentration of NaClO from 1% to 0.1%, hence treating the subgingival biofilm sequentially with TiBrush® + 0.1% NaClO + 0.2% CHX, a strong immediate as well as a prolonged bactericidal effect after 24 hours could still be seen (see in Figure 4b).

Thus, further experiments confirm the hypothesis that a sequential usage of immediate and substantive bactericidal and/or bacteriostatic agents leads to substantially no CFU counts after 24 h on a SLA® surface.

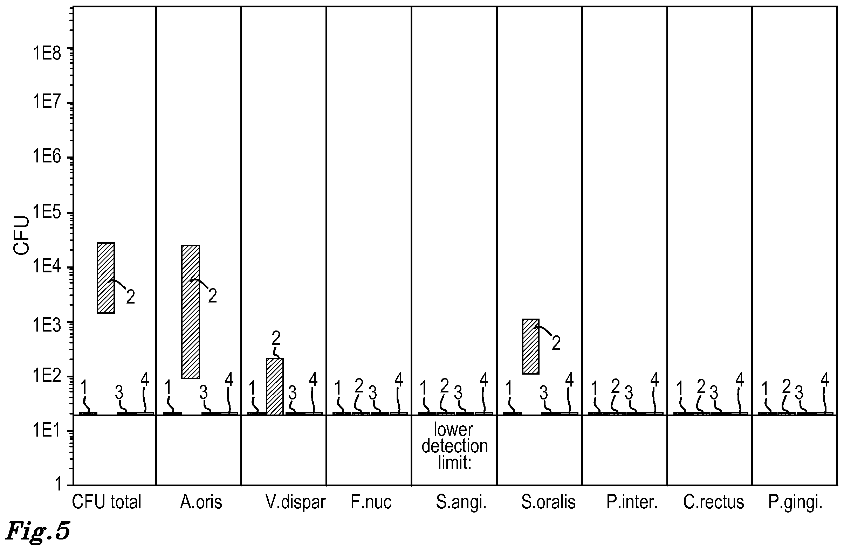

Extending the regrowth time from 24 hours to 48 hours, the sustainable bactericidal effect was still observed after treating the subgingival biofilm with TiBrush® + 0.1% NaClO + 0.2% CHX. After 72 hours regrowth, CFU values of 10⁵, four log steps below the control, were observed, (see figure 5c).

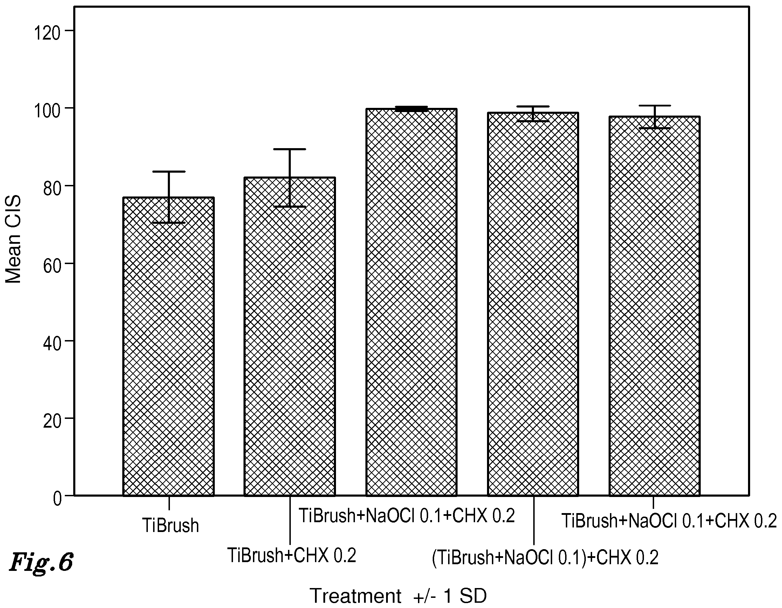

As can further be seen e.g. in figure 6, analysis on individual bacteria CFU count show that it is even possible to employ a 0.1% NaClO solution, if used according to the herein described cleaning procedure, i.e. in sequence with CHX. Figure 6 presents a summary of the test results from the subgingival biofilm models employed. The CFU count is detected after sequential cleaning with TiBrush®, NaClO solution 1% and CHX solution 0.2%. Using only TiBrush® and a NaClO solution 0.1%, regrowth is seen within some bacterial strains. See also figure 18.

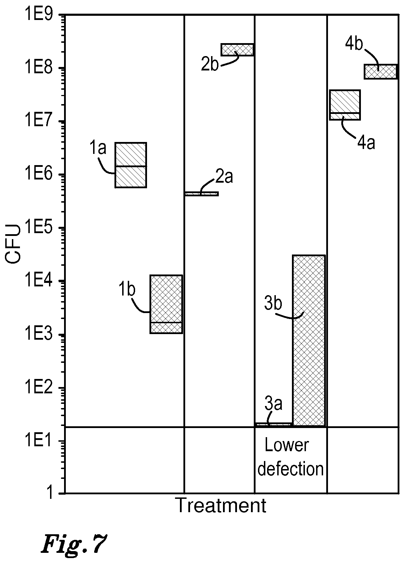

The above mentioned findings are further confirmed by comparison of data on Clean Implant Surface (CIS) after treatment of Straumann SLA® discs with TiBrush® and cleaning agents in a human in-vivo splint model is shown in figure 7. The use of TiBrush® + NaClO + CHX, at concentrations indicated below, resulted in a significant higher biofilm removal than obtained by using TiBrush® alone or TiBrush® + 0.2% CHX. Both the sequential treatments TiBrush® + 0.1% NaOCl + 0.2% CHX and TiBrush® + 1% NaOCI + 0.2% CHX as well as the treatment with combined application of TiBrush® and 0.1% NaOCI followed by 0.2% CHX showed a nearly complete clean surface, as seen in figure 7.

In summary, the above described effect of sustainably removing and/or disrupting a pathogenic biofilm is in particular observed in subgingival biofilms, which are in the field of the art acknowledged to be particularly hard to destroy, kill, remove and/or disrupt. Thus, the present invention in one embodiment, for the first time, discloses a method and a kit of parts for use in treating a periodontal disease, such as periimplantitis, caused by microorganisms that colonize a tooth and/or implant surface and that mainly reside in a subgingival biofilm.

### Chlorhexidine

The presently disclosed method and/or kit of parts for use in treating periodontal diseases such as periimplantitis, gingivitis, periodontitis or periimplant mucositis comprises a cleaning and/or disinfecting agent with a sustainable bactericidal effect (substantive bactericidal effect), such as chlorhexidine (CHX).

Chlorhexidine is a chemical antiseptic and it is effective on both Gram-positive and Gram-negative bacteria, although it is less effective with some Gram-negative bacteria. It has both bactericidal and bacteriostatic mechanisms of action, the mechanism of action being membrane disruption. Chlorhexidine is harmful in high concentrations, but is used safely in low concentrations in many products, such as mouthwash and contact lens solutions.

Chlorhexidine (CHX) is a synthetic cationic bis-guanide that consists of two symmetric 4-chlorophenyl rings and two biguanide groups connected by a central hexamethylene chain. CHX is a positively charged hydrophobic and lipophilic molecule that interacts with phospholipids and lipopolysaccharides on the cell membrane of bacteria and then enters the cell through some type of active or passive transport mechanism. Its efficacy is because of the interaction of the positive charge of the molecule and the negatively charged phosphate groups on microbial cell walls, thereby altering the cells' osmotic equilibrium. This increases the permeability of the cell wall, which allows the CHX molecule to penetrate into the bacteria. CHX is a base and is stable as a salt. The most common oral preparation, CHX gluconate, is water-soluble and at physiologic pH, it readily dissociates and releases the positively charged CHX component.

Chlorhexidine (C₂₂H₃₀Cl₂N₁₀) is present in oral rinses and skin cleansers and in small quantities it is used as a preservative. It is often used as an active ingredient in mouthwash designed to reduce dental plaque and oral bacteria. It has been shown to have an immediate bactericidal action and a prolonged bacteriostatic action due to adsorption onto the pellicle-coated enamel surface.

Chlorhexidine gluconate has become recognized as an effective antimicrobial agent, and its use as a potential endodontic irrigant has been demonstrated in the last decade. It possesses a broad-spectrum antimicrobial action, substantivity, and a relative absence of toxicity that are desirable properties of an ideal root canal irrigant. However, a significant attribute that chlorhexidine gluconate is not known to possess is a tissue dissolving property.

Chlorhexidine is a potent antiseptic, which is widely used for chemical plaque control in the oral cavity. Aqueous solutions of 0.1 to 0.2% are recommended for that purpose, while 2% is the concentration of root canal irrigating solutions usually found in the endodontic literature. It is commonly held that chlorhexidine would be less caustic than sodium hypochlorite. However, a 2% chlorhexidine solution is irritating to the skin.

In the present context, the term chlorhexidine is intended to include chlorhexidine and chlorhexidine gluconate, as well as chlorhexidine digluconate. Further, the abbreviation "CHX" is used interchangeably for Chlorhexidine and Chlorhexidine gluconate or digluconate, each and all of which can be comprised in the present treatment and or kit of parts.

In the present case, a solution of CHX comprises typically water and/or acetic acid as a solvent(s).

The concentration of the CHX solution applied in the present treatment and/or kit of parts for use according to the present invention is in a range of from 0.01 to 1 weight %.

For use in an application regimen as described herein, i.e. after an initial application of NaClO, the CHX solution applied in the present treatment and/or kit of parts is used at a particularly low concentration, i.e. in a range of from about 0.01-1 weight%. Typically, a CHX solution employed herein will have a concentration of about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.3, 0.4, 0.5, 0.6, 0.7, 0.8, 0.9, or 1.0 weight%. Typically, the concentration of the CHX solution applied in the present treatment and/or kit of parts is no higher than approximately 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.3, 0.4, 0.5, 0.6, 0.7, 0.8, 0.9, or 1.0 weight%.

What is more, the CHX solution employed in the present treatment and/or kit of parts can additionally comprise a coloring agent.

In addition, the CHX solution employed in the present treatment and/or kit of parts can additionally comprise a viscosity modifier, such as but not limited to the group consisting of PGA, hydroxymethylcellulose, hydroxyethylcellulose, carboxymethylcellulose, hydrophilic fumed silica, smectite clay xantan, gum, and magnesium aluminum silicate.

### Sodium hypochlorite

The presently disclosed method and/or kit of parts for use in treating periodontal diseases, such as periimplantitis, gingivitis, periodontitis or periimplant mucositis, comprises a cleaning and/or disinfecting agent with an immediate bactericidal effect, such as NaClO. Sodium hypochlorite is a chemical compound with the formula NaClO. Sodium hypochlorite solution, commonly known as bleach or Clorox, is frequently used as a disinfectant or a bleaching agent.

In the present context, NaClO solution is comprised in the present treatment and or kit of parts.

In the present case, NaClO is typically solved in water.

The concentration of the NaClO solution applied in the present treatment and/or kit of parts for use according to the present invention is in a range 0.01-1 weight%, such as between 0.05-1 weight%, such as between 0.01-0.1 weight%, such as between 0.01-0.05 weight%, such as between 0.05-0.1 weight%, such as between 0.1-1 weight%, such as between 0.1-0.2 weight%, such as between 0.1-0.3 weight%, such as between 0.1-0.5 weight%, or such as between 0.1-0.7 weight%.

For use in an application regimen as described herein, i.e. followed by an application of CHX, the NaClO solution applied in the present treatment and/or kit of parts can be used at a particularly low concentration, i.e. in a range of from about 0.01-1 weight%.

Typically, a NaClO solution employed herein will be of about 0.05, 0.06, 0.07, 0.08, 0.09, 0.1, 0.2, 0.3, 0.4, 0.5, 0.6, 0.7, 0.8, 0.9, or 1.0 weight%. Typically, the concentration of the NaClO solution applied in the present treatment and/or kit of parts is no higher than approximately 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.3, 0.4, 0.5, 0.6, 0.7, 0.8, 0.9, or 1.0 weight%. What is more, the NaClO solution employed in the present treatment and/or kit of parts can additionally comprise a coloring agent.

In addition, the NaClO solution employed in the present treatment and/or kit of parts can additionally comprise a viscosity modifier, such as but not limited to the group consisting of PGA, hydroxymethylcellulose, hydroxyethylcellulose, carboxymethylcellulose, hydrophilic fumed silica, smectite clay xantan, gum, and magnesium aluminum silicate.

### Cleaning and/or disinfecting agents

It is presently envisioned to use at least two cleaning and/or disinfecting agents for manufacturing a kit of parts for use in treating a periodontal disease selected from: a) NaClO solution, and b) CHX solution, and an instruction describing the use of the above two agents in a given sequential order of first using agent a) and thereafter using agent b), and optionally rinsing the site of microbial infection to be treated both before, in between, during and/or after the application of the above cleaning agents.

The herein described sequential use of the at least two cleaning and/or disinfecting agents leads to a substantive removal, destruction, killing and/or disruption of a pathogenic biofilm at a site of microbial infection in a patient suffering from a periodontal disease either supragingival and/or subgingival, at the same time effectively and sustainably inhibiting, obstructing, impeding and/or preventing the growth and/or regrowth of a pathogenic biofilm at the same site of microbial infection in said patient.

In a use as described herein, the NaClO solution has a concentration of from 0.01 to 1.0 weight%, such as 0.1 weight%, such as 1 weight%, and the CHX solution has a concentration of from 0.01 to 1.0 weight%, such as 0.2 weight%.

The cleaning and/or disinfecting agent(s) comprised in the present treatment and/or kit of parts for use according to the present invention may also comprise a pharmaceutically acceptable carrier and/or a pharmaceutically acceptable diluent and/or a pharmaceutically acceptable excipient. The cleaning and/or disinfecting agent(s) may also comprise one or more additional compound(s) such as one or more antimicrobial compound(s) and one or more other pharmaceutically active compound(s).

The antimicrobial and antibiotic may be selected from antibacterial or antifungal compounds such as, but not limited to, tobramycin, ciprofloxaxine, colistin, silver compounds, amino glycosides, macrolides, fluoroquinolones, ceftazimides, tetracyclines, sulfonamides, beta-lactams, oxazolidiones, anitimicrobal peptides, xylitol, framycetin, fusidic acid, nitrofural, phenylmercuric nitrate, benxododecinium, triclosan, cetylpyridinium, aluminium chlorohydrate, povidoneiodine, cloauinol, benzalkonium, chlorohexidine, iodoform, hypochloride acid, tetracyclin hydrochloride, ampicillin, piperacillin, gentamycin, dibekacin, kanendomycin, lividomycin, tobramycin, amikacin, fradiomycin, sisomicin, tetracyclin, oxytetracyclin, rolitetracyclin, doxycyclin, ampicillin, piperacillin, ticarcillin, cefalotin, cefapirin, cefaloridine, cefaclor, cefalexin, cefroxadine, cefadroxil, cefamandole, cefotoam, cefroxime, cefotiam, cefotiam hexetil, cefuroxime axetil, cefdinir, cefditoren pivoxil, ceftazidime, cefpiramide, cefsulodin, cefinenoxime, cefpodoxime proxetil, cefpirome, cefozopran, cefepime, cefsulodin, cefinenoxime, cefinetazole, cefminox, cefoxitin, cefbuperazone, latamoxef, flomoxef, cefazolin, cefotaxime, cefoperazon, ceftizoxime, moxalactam, thienamycin, sulfazecin, azthreonam and their salts, griseofulvin, lankacidin, polyene-based antibiotics (e.g., amphotericin B, nystatin, trichomycin); griseofulvin, pyrrolnitrin, and the like; cytosine metabilism antagonists (e.g., flucytosine); imidazole derivatives (e.g., econazole, clotrimazole, miconazole nitrate, bifonazole, croconazole); triazole derivatives (e.g., fluconazole, itraconazole, azole-based compounds, e.g., [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl-3-(2H,4H)-1,2,4-triazolone); thiocarbamic acid derivatives (e.g., trinaphthol); echinocandin-based derivatives (e.g., caspofamgine, FK-463, V-Echinocadin), cetylpyridinklorid, bacimycin, brulidine, ethanol or quaternary ammonium compounds, cephalexin, augmentin , ampicillin-sulbactam, duricef, dicloxacillin, ticarcillin-clavulanic acid, piperacillin-tazobactam, cefazolin, cefotetan, cefoxitin, imipenem, terbinafin, fluconazol, ketoconazole, mikonazolnitrat, chlotrimazole, amorolfin and econazolnitrate and combinations thereof.

Examples of, but not limited to, pharmaceutically acceptable compounds are pain-killing agents such Naproxen, Ibuprofen, Ketoprofen, Fenoprofen, Flurbiprofen, Dexibuprofen or Tiaprofenic acid, Diclofenac, Alclofenac, Fenclofenac, Etodolac, Aceclofenac, Sulindac or Indomethacin, Ketorolac or Tolmetin, Mefenamic acid, Acetyl salicylic acid (Aspirin), Salicylic acid or Diffunisal, Phenylbutazone, Piroxicam, Tenooxicam, Meloxicam or Lornoxicam, Aminopyrene or antipyrene, Acetaminophen Phenacetin, Nabumeton, Celecoxib and Rofecoxib. Other examples of pharmaceutically acceptable compounds are non-steroidal antiinflammatory agents which include, but are not limited to, acetaminophen, fenasetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizol, tiaramide hydrochloride, zaltoprofen, gabexate mesilate, camostat mesilate, ulinastatin, colchicine, probenecid, sulfinpyrazone, benzbromarone, allopurinol, sodium gold thiomalate, sodium hyaluronate, sodium salicylate, morphine hydrochloride, salicylic acid, atropine, scopolamine, morphine, pethidine, levorphanol, ketoprofen, naproxen, oxymorphone, and their salts. Other examples are steroidal agents including, but not limited to, dexamethasone, hexestrol, methimazole, betamethasone, triamcinolone, triamcinolone acetonide, fluorocinonide, fluorocinolone acetonide, prednisolone, methylprednisolone, cortisone acetate, hydrocortisone, fluorometholone, beclometasone dipropionate, estriol, and the like. More examples of pharmaceutical compounds are, cocaine hydrochloride, procaine hydrochlodie, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine, and the like, or other systemic, inhalation, or intravenous anesthetics. Aslo diabetes agents may be used including, but not limited to, actos, lodiglitazon, kinedak, penfill, humalin, euglucon, glimicron, daonil, novolin, monotard, insulins, glucobay, dimelin, rastinon, bacilcon, deamelin S, Iszilins]; hypothyroidism treating agent [dried thyroid gland (thyreoid), levothyroxine sodium (thyradin S), liothyronidin sodium (thyronine, thyromin), and the like. Combinations thereof may also be used.

The cleaning and/or disinfecting agent(s) comprised in the present method of treatment and/or kit of parts for use according to the present invention may be prepared according to known methods. The skilled person is aware of a vast variety of suitable vehicles that may be used for the agent(s) of the present invention. Furthermore, a cleaning and/or a disinfecting solution comprised in the present treatment and/or kit of parts for use according to the present invention may be sterile, i.e. it is essentially free of microorganism such as fungi, bacteria and/or virus. A sterile solution may for example be achieved by using sterile components and/or by sterilizing the agent after its preparation.

The cleaning and/or disinfecting agent(s) comprised in the present method of treatment and/or kit of parts for use according to the present invention may be applied once or repeatedly.

Furthermore, the cleaning and/or disinfecting agents may be applied for a period of 0.1, 0.2, 0.3, 0.4, 0.5, 0.6, 0.7, 0.8, 0.9, 1, 2, 3, 4, 5, 10 or 15 minutes to 1 hour, such as from 10 seconds to 15 minutes, such as from 10 seconds to 1 minute, such as from 10 seconds to 5 minutes, such as from 20 seconds to 15 minutes, such as from 1 minute to 5 minutes, such as from 2 minutes to 10 minutes, such as from 20 minutes to 55 minutes such as from 20 to 30 minutes. In one embodiment, at least one of the cleaning agent(s) are applied as rinse solution(s). In such a use, the cleaning agent as defined herein may be applied for a period of less than 0.5-5 minutes, and may instantly or after a short period of time be followed by another rinse with a sterile solution, such as saline, or immediately be followed by a rinse with the following cleaning agent.

One skilled in the art will readily appreciate that the administration duration and dosage of the cleaning agents as defined herein may be determined or adjusted based on the age, body weight, general condition, sex, diet, the severity of the infection, and/or degree of inflammation and/or infection associated with the periodontal disease. The treatment can be given repeatedly, depending upon the effect of the initial treatment regimen.

The cleaning and/or disinfecting agent(s) comprised in the treatment and/or kit of parts for use according to the present invention as described herein may be provided in any suitable form for administration to a surgically opened wound. For example, said agent(s) may be comprised in a composition for topical and/or local administration. Additionally, the cleaning and/or disinfecting agent(s) may be applied by using a delivery system. Said system may comprise a container, a syringe, a gel, a sponge, a foam and/or dressing. Also, the cleaning agent(s) may be applied by using any suitable sterile applicator or other sterile method known in the art.

The cleaning and/or disinfecting agent(s) comprised in the treatment and/or kit of parts for use according to the present invention as described herein may be formulated as a sequential release formulation for treating a periodontal disease, selected from the group consisting of periimplantitis, gingivitis, periodontitis and periimplant mucositis, in a patient suffering from a periodontal disease, comprising at least two cleaning agents selected from a sodium hypochlorite (NaClO) solution and a chlorhexidine (CHX) solution, wherein said agents are released sequentially at the site of administration. In such a sequential release, the NaClO solution typically has a concentration of from 0.01 to 1.0 weight%, such as 0.1 or 1 weight%, and the CHX solution has a concentration of from 0.01 to 1.0 weight%, such as 0.2 weight%.

### The mechanical cleaning and/or debridement tool

The presently disclosed method and/or kit of parts for use in treating a periodontal disease, such as periimplantitis, gingivitis, periodontitis or periimplant mucositis, comprises a method for sustainably removing, destroying, killing and/or disrupting a pathogenic biofilm at a site of microbial infection in a patient suffering from a periodontal disease, in one embodiment characterized by using a mechanical cleaning and/or debridement tool in combination with using first at least one cleaning and/or disinfecting agent with an immediate bactericidal effect, and thereafter using at least one cleaning and/or disinfecting agent with a sustainable bactericidal effect (substantive bactericidal effect).

Typically, such a mechanical cleaning and/or debridement tool is selected from the non-limiting group consisting of a brush, a cuvette, a spatula and an ultrasonic device.

The present invention therefore in one embodiment provides a method of treatment and/or a kit of parts for use according to the present invention comprising at least one medical implant cleaning and/or debridement brush, comprising a cleaning section comprising titanium and/or titanium alloy bristle(s) for cleaning and/or debriding the surface of a medical implant.

The brush in accordance with the present invention must be suitable for cleaning and/or debriding the surface of a medical implant. In the context of the present invention, this means that the brush can be utilized for removing contaminants e.g. bacterial biofilm, debris, calculus, fibrous tissue, concrements, microbes, unwanted tissue, cells and cell residues, scar tissue, and/or necrotic tissue from the implant.

Any brush designed for cleaning and/or debriding medical implants can also be used by the dentist or surgeon to clean and/or debride both hard and soft tissue, should this be considered necessary.

The material(s) and dimensions of the brush and its different parts are selected so as to enable the brush's use with a medical implant, such as a dental implant or an implant for CMF and/or orthopedic applications. This means that the length and diameter of the brush must be selected to enable cleaning of the desired implant. For example, when the brush is designed for use with a dental implant, it cannot be too long or its diameter too wide that it cannot comfortably be manipulated within the patient's mouth and fit within the bone cavity around the implant. Further, the materials used for the bristles as well as for other parts of the brush are typically biocompactible and have properties which exert limited damage to the surfaces that are contacted by the brush.

The bristle(s) of the brush preferably consist of titanium and/or a titanium alloy. The term "alloy" is herein intended to mean a metallic material containing a base metal and at least one alloying component. The term "base metal" is herein intended to mean the metal being the primary constituent of the alloy and the term "alloying component" is intended to mean a component added to the base metal in order to form the alloy. Thus, the term "titanium alloy" is intended to mean an alloy comprising titanium as base metal and at least one alloying component.

Thus, the bristle(s) may consist of pure, i.e. unalloyed, titanium. For example, the bristle(s) may consist of titanium selected from the group consisting of: titanium of grade 1, titanium of grade 2, titanium of grade 3, titanium of grade 4 and titanium of grade 5, according to ASTM F67. These types of titanium are sometimes also denoted as "commercially pure" titanium.

At least some of the bristle(s) however preferably consist of a titanium alloy, whereby the titanium alloy comprises titanium as base metal and at least one alloying component selected from the group consisting of nickel, zirconium, tantalum, hafnium, niobium, aluminium, vanadium, molybdenum, chrome, cobalt, magnesium, iron, gold, silver, copper, mercury, tin and zinc.

In accordance with one embodiment, the bristle(s) may consist of a titanium alloy, whereby the titanium alloy comprises titanium as base metal, and aluminum and vanadium as alloying components. One preferred example of such a titanium alloy comprises about 94.5 weight% titanium, about 3 weight% aluminum and about 2.5 weight% vanadium.

The bristles may comprise different materials, in other words, individual bristles may comprise different materials in the same brush. In some embodiments not all of the bristles will consist of titanium or titanium alloy. For example, some nylon or polymer bristles may also be included in the brush. However, preferably all the bristles consist of titanium or titanium alloy; more preferably all of the bristles are formed of the same material, e.g. one of the above titanium alloys.

A brush suitable for use as the brush in the present invention is the twisted-in-wire brush disclosed in WO 2009/083281, to which reference is herewith made. In the present context, a twisted-in-wire brush as disclosed in WO 2009/083281 is used in the experimental section, which is presently sold under the trademark TiBrush®.

Another type of brush suitable for use in the present invention comprises a first main part which is a handle that is stiff, plastically deformable or elastically deformable and a second main part that is at least one cleaning element comprising a base part and one or several bristle(s), which can be in the form of loops, or a cam of spikes. The bristle loops in such a brush may be seen as a grooved strip that is wound around the base member.

An example of such a brush is disclosed in WO 2011/152789, to which reference is herewith made.

The brush disclosed herein may be in combination with a rotationally oscillating device for a plethora of medical and dental applications *in vivo* and *in vitro.*

The brush of the present invention may be utilized during surgery for cleaning of the surface of a medical implant after infection and/or bone resorption. For example, it may be utilized for cleaning the surface of a dental implant. Alternatively, or in addition, the brush may be used in order to remove e.g. a bacterial biofilm, debris, calculus, fibrous tissue, concrements, microbes, unwanted tissue, cells and cell residues, scar tissue, and/or necrotic tissue from the vicinity of the dental fixture prior to, or after, implantation. The brush may also be utilized for cleaning the surface of an abutment.

The brush as defined above in combination with a rotationally oscillating handpiece is advantageous to utilize for cleaning medical implants, in particular dental and/or CMF implants. It is advantageous to utilize for cleaning both "hard" metallic implants having relatively hard surfaces, such as implants comprising or consisting of steel, and "soft" implants having delicate surfaces, such as e.g. titanium, a titanium alloy, zirconium or a zirconium alloy. In addition, the brush is as well suited for use in the cleaning of non-metallic implants, such as those which comprise or consist of e.g. ceramic.

The titanium or titanium alloy of which the bristle(s) are made may be selected such that the hardness degree thereof exactly, or at least essentially, corresponds to the hardness degree of the implant surface to be cleaned. For example, in case the implant to be cleaned consists of pure titanium, pure titanium can be selected as the material of the bristle(s). Alternatively, the hardness of the bristles can be chosen to best match the hardness of the material from which the implant is made, e.g. zirconium or ceramics.

### Method

### Kit of parts

In one embodiment of the present invention, a kit of parts is disclosed for use in treating a periodontal disease, such as periimplantitis, gingivitis, periodontitis or periimplant mucositis, caused by microorganisms that colonize a tooth and/or implant surface at, above and/or below the gingival margin, and which reside in a biofilm, either subgingival and/or supragingival.

The present invention relates thus also to a kit of parts for use in sustainably removing, destroying, killing and/or disrupting a pathogenic biofilm at a site of microbial infection in a patient suffering from a periodontal disease, and comprises a) at least one cleaning and/or disinfecting agent with an immediate bactericidal effect, and b) at least one cleaning and/or disinfecting agent with a sustainable bactericidal effect (substantive bactericidal effect). Agents a) and b) are not identical. Optionally, a mechanical cleaning and/or debridement device is further comprised in said kit of parts. Optionally again, a description, such as but not limited to a written description, is comprised in the kit as well. In particular, the kit of parts disclosed herein for use according to the present invention comprises at least two cleaning and/or disinfecting agents for sustainably removing, destroying, killing and/or disrupting a pathogenic biofilm at a site of microbial infection in a patient suffering from a periodontal disease, selected from: a) NaClO solution, and b) CHX solution, and c) an instruction describing the use of the two cleaning agents in a given sequential order of first applying a), and thereafter applying b). The kit of parts as defined herein, optionally comprises one or more rinsing and/or cleaning agents to be used for rinsing the site of the microbial infection before and/or in between and/or after the application of the cleaning agents. Additionally, the kit of parts as disclosed herein may optionally also comprise a cleaning and/or debridement tool, such as a suitable brush. The kit of parts for use according to the present invention as defined herein may comprise a titanium bristled brush, which can be used before or together with solution a) and/or b). Furthermore, the kit of parts as for use according to the present invention defined herein may also comprise at least one or more cleaning and/or rinsing agent(s) to be administered after solution a) and/or b). The cleaning and/or rinsing agents may be selected from the group consisting) of NaCl and H₂O.

In one embodiment, a kit of parts for use according to the present invention as defined herein comprises instructions, which can be, but are not limited to, written instructions, describing a sequential administration to a site of microbial infection in a patient in such a way that the sodium hypochlorite (NaClO) solution is to be administered before the chlorhexidine (CHX) solution.

The NaClO solution comprised in the kit of parts for use according to the present invention as disclosed herein may have a concentration of from 0.01 to 1.0 weight%, such as 0.1, or 1 weight%, and the CHX solution has a concentration of from 0.01 to 1.0 weight%, such as 0.2 weight%.

The kit of parts for use according to the present invention as defined herein optionally comprises a mechanical cleaning and/or debridement tool, such as a brush designed for cleaning and/or debriding medical implants. The tool is used by the dentist or surgeon to clean and/or debride both hard and soft tissue, should this be considered necessary.

The kit of parts for use according to the present invention described herein is suitable for sustainably removing and/or destroying and/or disrupting a pathogenic biofilm at a site of microbial infection in a patient suffering from a periodontal disease, which comprises surgically assessing the infected site and employing a mechanical cleaning and/or debridement tool and applying first at least one cleaning and/or disinfecting agent with an immediate bactericidal effect, and thereafter applying at least one cleaning and/or disinfecting agent with a sustainable bactericidal effect (substantive bactericidal effect).

A kit of parts as defined herein may be for use in treating a subgingival and/or supragingival microbial infection in a patient suffering from e.g. periimplantitis, gingivitis, periodontitis and/or periimplant mucositis, the use comprises the following steps: a) cleaning and/or disinfecting the site of microbial infection with a sodium hypochlorite (NaClO) solution; and thereafter b) cleaning and/or disinfecting the site of microbial infection with a chlorhexidine (CHX) solution.

A kit of parts as herein disclosed may be for use in preparing the surface for regenerative treatment.

In addition, the kit of parts may be for use in *in vitro* uses which include, but are not limited to, the cleaning of parts of dental implants, such as abutments, before repositioning in a subject.

Furthermore, the kit of parts as disclosed herein may be for use in a method for treating a periodontal disease in a patient suffering from e.g. periimplantitis, gingivitis, periodontitis and/or periimplant mucositis, which method is characterized by long term and/or permanent destruction, disruption and/or removal of a pathogenic biofilm at a site of microbial infection in a patient suffering from a periodontal disease. Accordingly, said kit of parts facilitates the effective and substantive removal and/or disruption and/or killing of a subgingival biofilm. In particular again, said method is especially effective for killing *P*. *gingivalis, B. forsythymus,* and/or *T*. *denticola* in said biofilm.

A brush which may be comprised in the kit of parts for use according to the present invention as defined herein is the twisted-in-wire brush disclosed in WO 2009/083281, to which reference is herewith made.

Another type of brush which may be comprised in a kit of parts for use according to the present invention as defined herein comprises a first main part which is a handle that is stiff, plastically deformable or elastically deformable and a second main part that is at least one cleaning element comprising a base part and one or several bristle(s), which can be in the form of loops, or a cam of spikes. The bristle loops in such a brush may be seen as a grooved strip that is wound around the base member. An example of such a brush is disclosed in WO 2011/152789, to which reference is herewith made.

In one embodiment, the kit of parts as for use according to the present invention disclosed herein is provided as a package comprising a container comprising a sodium hypochlorite (NaClO) solution, a container comprising a chlorhexidine (CHX) solution; and instructions of use describing administering the content of the containers in a given sequential order of first administering the content of container a) and thereafter the content of container b) to a site of microbial infection in a patient in need thereof.

According to the present invention, such a package comprises at least one container selected from the group consisting of a syringe, a sponge, a dressing and a gel.

### Other embodiments

It is to be understood that while the present invention has been described in conjunction with the detailed description thereof, the foregoing description is intended to illustrate and not limit the scope of the invention, which is defined by the scope of the appended claims. Other aspects, advantages, and modifications are within the scope of the following claims.

### Experimental section

The present invention is further illustrated by the following non-limiting experiments.

### Experiment 1

### Evaluation of biofilm decontamination with various antimicrobial solutions

### Tested in a 70/30 supragingival biofilm model

For a detailed description of the biofilm model used in this experiment, see e.g. Guggenheim et al., 2004, 2001a; and Shapiro et al., 2002 and Guggenheim et al., 2009.

### STRAINS:

OMZ 918, *Streptococcus mutans*
OMZ 493, *Veilonella dispar*
OMZ 598, *Fusobacterium nucleatum*
OMZ 607, *Streptococcus oralis*
OMZ 745, *Actinomyces oris*
OMZ 110, *Candida albicans*

### TEST SOLUTIONS:

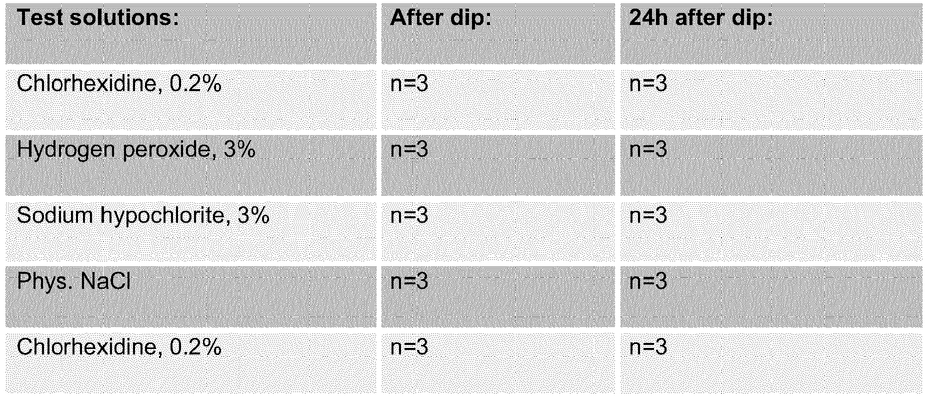

### RESULTS:

As can be seen in figure 7, compared to the saline control, chlorhexidine (CHX), and hydrogen peroxide (H₂O₂), had very modest immediate decontaminating effect. In contrast, the effect immediately after exposing the biofilms to sodium hypochlorite was very impressive. The recovery of the microorganisms after 24 h showed a distinctly different picture. H₂O₂ had no or minute lasting biofilm inhibiting action. CHX still had a strong lasting effect.

### SUMMARY:

The present biofilm study may be summarized as follows. While all agents tested immediately after application had albeit a differently strong decontamination effect, only the lasting effect after 24 h provides information on how valuable their use in daily practice will be. Under these premises only CHX had a lasting effect, while all the other agents should no longer be considered as promising agents for use in periimplantitis treatment.

### Experiment 2

### Evaluation of biofilm decontamination with various antimicrobial solutions

### Tested in a 70/30 supragingival biofilm model

For a detailed description of the biofilm model used in this experiment, see e.g. Guggenheim et al., 2004, 2001a; and Shapiro et al., 2002 and Guggenheim et al., 2009.

### STRAINS:

OMZ 918, *Streptococcus mutans*
OMZ 493, *Veilonella dispar*
OMZ 598, *Fusobacterium nucleatum*
OMZ 607, *Streptococcus oralis*
OMZ 745, *Actinomyces oris*
OMZ 110, *Candida albicans*

### TEST SOLUTIONS:

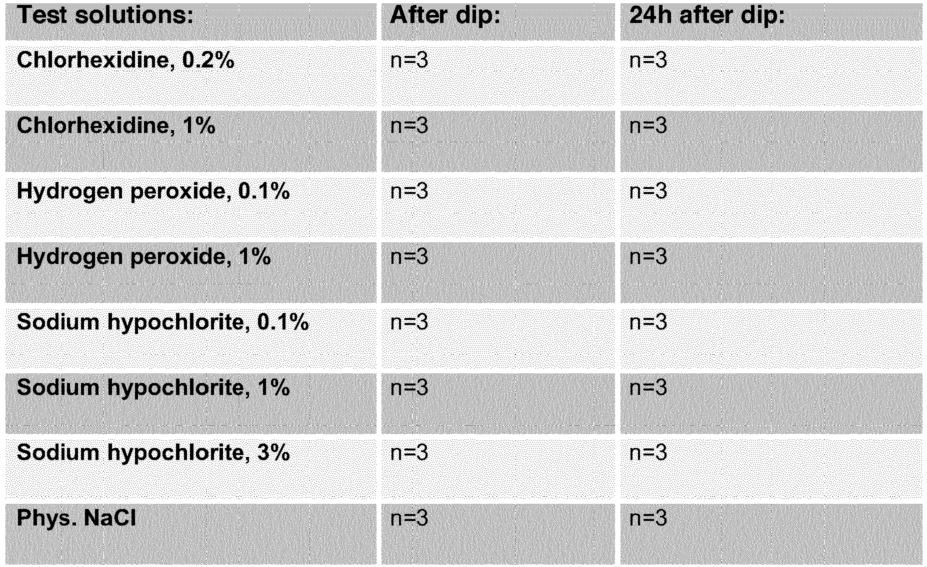

### RESULTS:

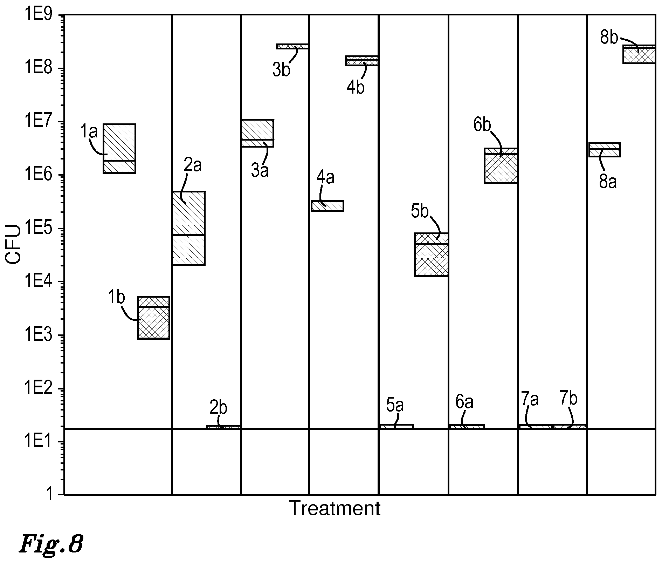

As can be seen in figure 8, the results of this experiment show the good reproducibility of the biofilm test. With regard to test solutions already applied in the previous biofilm study (experiment 1), the results are most coherent, this is also evident comparing the initial inocula densities in both experiments.

The present experiment therefore confirms overall the previous results obtained. The 0.2% CHX solution had a low immediate effect, but after 24, the known sustained substantivity of CHX reduced the biofilm microbiota by almost 6 log steps. No surprise that the more concentrated 1.0% CHX solution had even a better efficacy at both time points eradicating totally the biofilm microbiota after 24 h.

The hydrogen peroxide solutions in both concentrations had a low decontaminating action immediately after application and after 24h, the microbiotes had re-established to the level of the controls. The sodium hypochlorite solutions in all concentrations tested had a very strong immediate effect lowering the biofilm microbiota below the detection level. However, after 24h, the bacteria treated with the 0.1 and 1.0% concentrations, had in part recovered. The observation that the 1.0% concentration after 24h was less effective than the 0.1% preparation is a result that can be explained by an outlier in the later triplicates. In contrast the 3.0% sodium hypochlorite solution showed to be a very good killer, leaving no detectable microorganisms on the discs at both time points.

### SUMMARY:

In conclusion, having a product decontaminating implant surfaces in approx. 3.0% sodium hypochlorite and 1.0% CHX seem to be the best choices among the compounds so far tested.

### Experiment 3

### Evaluation of subgingival biofilm decontamination with various antimicrobial solutions

### tested in a subgingival biofilm model

For a detailed description of the biofilm model used in this experiment, see e.g. Guggenheim et al., 2004, 2001a; and Shapiro et al., 2002 and Guggenheim et al., 2009.

### STRAINS:

OMZ 278, *Prevotella intermedia*
OMZ 493, *Veilonella dispar*
OMZ 598, *Fusobacterium nucleatum*
OMZ 607, *Streptococcus oralis*
OMZ 661, *Treponema denticola*
OMZ 698, *Campylobacter rectus*
OMZ 745, *Actinomyces oris*
OMZ 871, *Streptococcus anginosus*
OMZ 925, *Porphyromonas gingivalis*
OMZ 1047, *Tannerella forsythia*

### TEST SOLUTIONS:

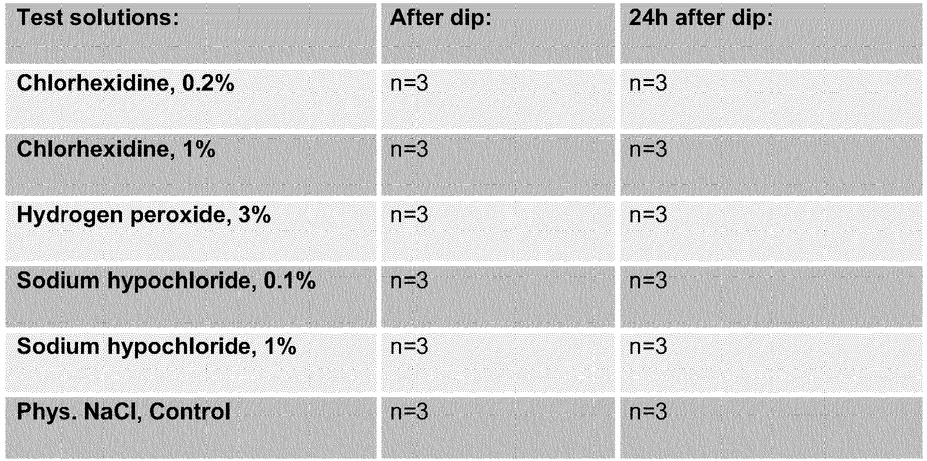

### RESULTS:

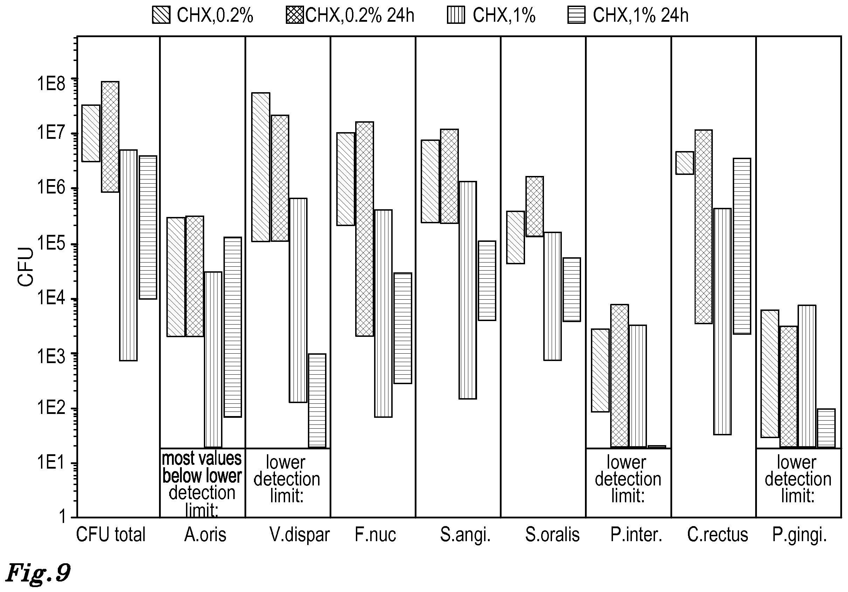

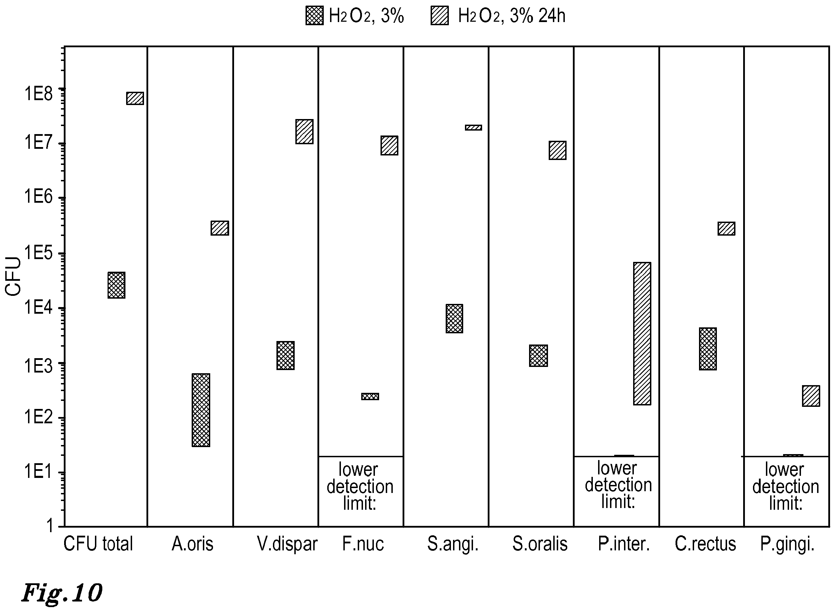

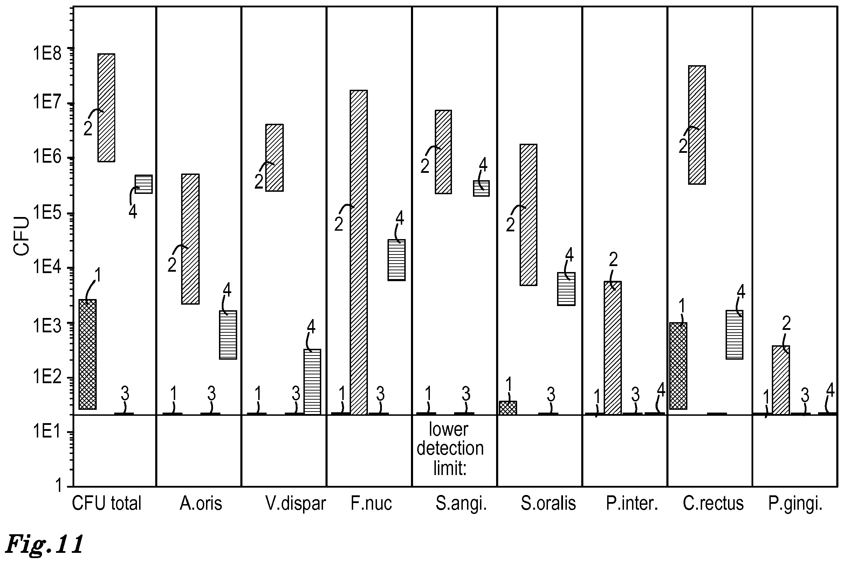

The purpose of the present biofilm experiment was to assess the efficacy of various antimicrobial solutions on a subgingival microbiota associated with periodontitis and implantitis. The antimicrobial effect was evaluated immediately and 24 h after a 1min exposure to the test solutions to biofilms grown on titanium discs. The quantity of total colony forming units was evaluated and also the effect on individual members of the biofilm consortium, as can be seen in figures 9-13.

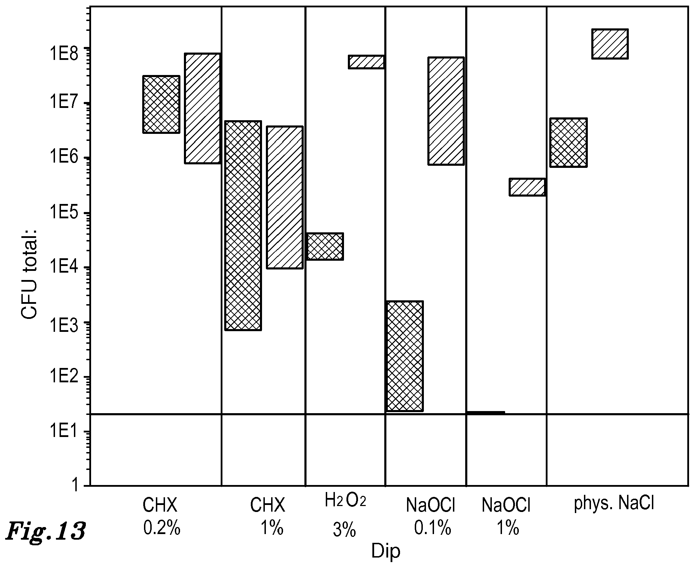

The overview in fig.13, where the effect of the different test solutions on total CFU is plotted, allows the best access to the results. The fully colored bars reflect the immediate effect after a 1min exposure to the antimicrobial solution; the hatched bars the sustained effect after 24 h. It is evident that all test solutions with the exception of 0.2% CHX reduced the microbiota on the biofilms compared with the saline controls to a different extent immediately after exposure. While 1% CHX, and 3% H₂O₂ had only a limited effect, NaClO in both concentrations reduced the microbiota near or to the detection limit.

However, after 24 h, none of the tested antimicrobial solutions had a strong sustained effect on the total CFU.

CHX 1% and NaClO 1% suppressed the microbiota less than 3 log steps. All the other test solutions could not inhibit biofilm re-growth and almost reached the level of the saline control group. One can conclude that a sole 1 min application is not sufficient to reduce the biofilm microbiota that could be of interest in a clinical application.

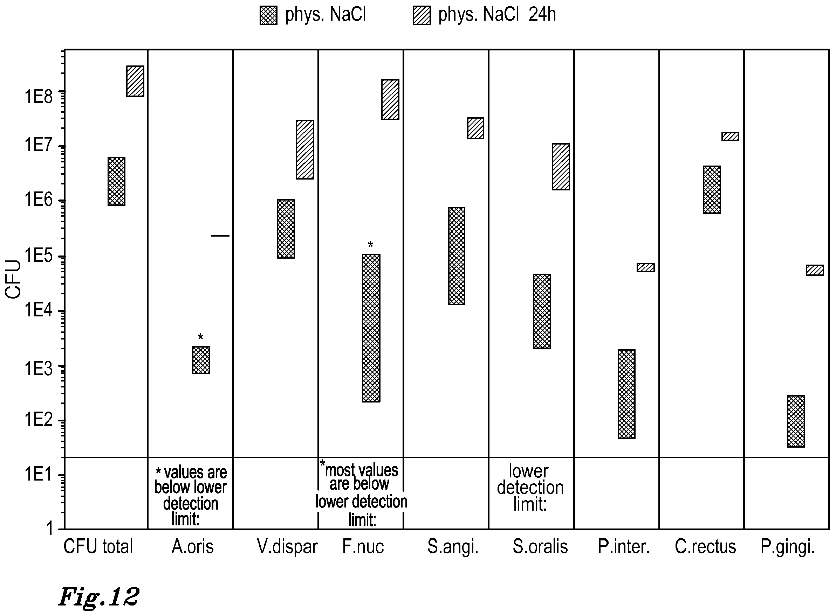

With regard to individual strains, the saline control shows that with the exception of *Tannerella forsythiae* and *Treponema denticola,* which could not be cultured, all other bacterial species could colonize in the biofilms (fig. 12). All species increased in numbers within 24 h following the 11 min treatment with saline. Following the pattern described above for total CFU, 1% CHX and 3% H₂O₂ had only a limited effect; NaClO in both concentrations reduced all species near or to the detection limit (figures 9, 10 and 11). With the exception of *P. intermedia* and, *P. gingivalis,* treated with CHX 1% and both concentrations of NaClO, all bacterial species were able to re-grow in the following 24 h and showed no sustained effect that could be of clinical importance. It is questionable, whether the eradication of the potentially pathogenic *P. intermedia* and *P. gingivalis* would persist, if the test period would be extended over 24 h.

### SUMMARY:

Therefore, it is concluded, that a lasting effect on the subgingival microbiota can only be achieved, if the treatment duration is substantially increased and/or is repeated within short intervals.

### Experiment 4

### Evaluation of subgingival biofilm decontamination with various antimicrobial solutions

### tested in a subgingival biofilm model

For a detailed description of the biofilm model used in this experiment, see e.g. Guggenheim et al., 2004, 2001a; and Shapiro et al., 2002 and Guggenheim et al., 2009.

### STRAINS:

OMZ 278, *Prevotella intermedia*
OMZ 493, *Veilonella dispar*
OMZ 598, *Fusobacterium nucleatum*
OMZ 607, *Streptococcus oralis*
OMZ 661, *Treponema denticola*
OMZ 698, *Campylobacter rectus*
OMZ 745, *Actinomyces oris*
OMZ 871, *Streptococcus anginosus*
OMZ 925, *Porphyromonas gingivalis*
OMZ 1047, *Tannerella forsythia*

### TEST SOLUTIONS:

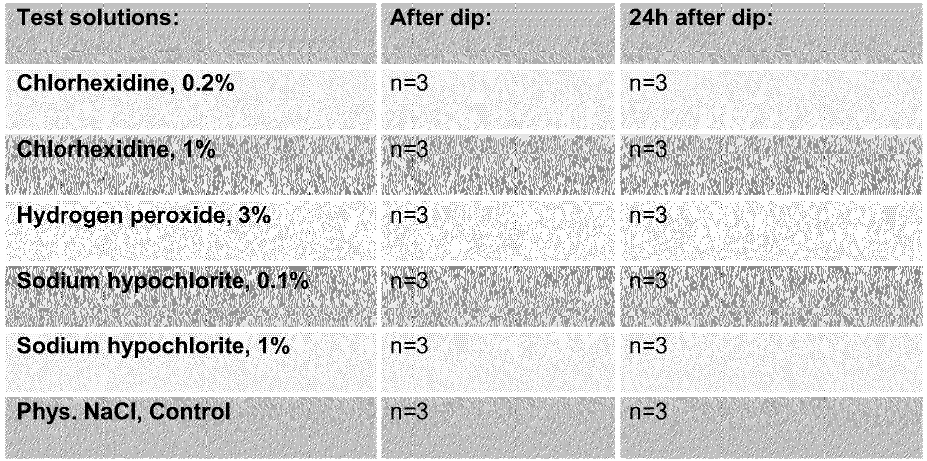

### RESULTS:

The subgingival biofilm experiments included 8 treatments that were split into two parts including 4 treatments each because of the demanding requirements of the test design in particular with regard to timing. To be able to compare the results of the 2 experiments, the NaCl control was repeated in both experiments.

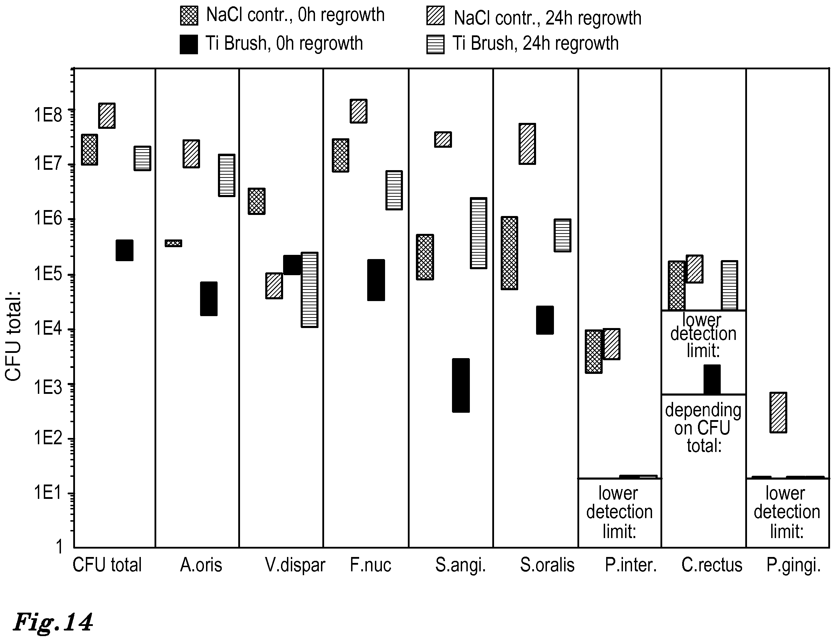

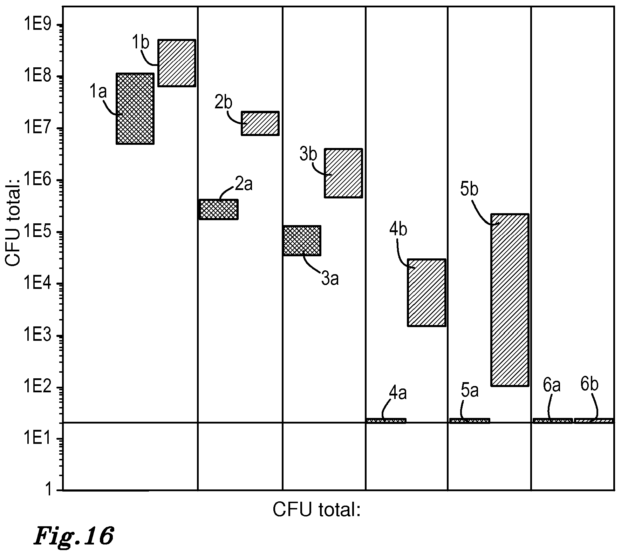

An overview of the results of both experiments is provided in figure 16. The mean values of the controls in the two experiments fluctuated within one log step allowing a direct comparison of all treatments in the 2 experiments. The titanium discs were colonized immediately after rinsing with saline by 4.5 E7 CFU and slightly increased after 24 h to 3.4 E8 CFU. In comparison to the control, the TI brush alone without antimicrobial treatment immediately after brushing reduced the number of bacteria by = 2 log steps. After 24 h regrowth by = 1.5 log steps was observed. When in addition 0.2% CHX was applied, the immediate effect was small and after 24h regrowth by = 1 log step was observed. A very strong immediate effect of brushing in combination with 0.1% NaClO solution was evident. Here the biofilm microbiota was reduced to the detection limit but increased after 24h by = 3 log steps. An increase of the NaClO solution concentration in the rinsing solution to 1% reduced the regrowth further, but with a great variation (see figure 14).

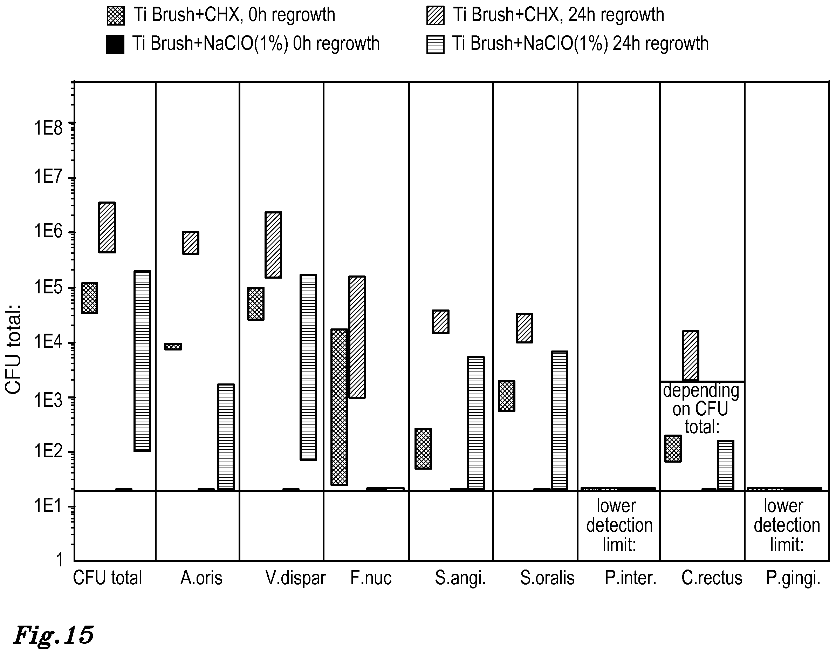

The best and impressing result was achieved by TI brushing and a combination of rinsing with 1% NaClO solution followed by a rise with 0.2% CHX. Here the biofilm microbiota was reduced immediately after treatment and also 24h later below detection limit (see figure 15).

### SUMMARY:

Analyzing the effect of these treatments on single species, there could be much to be commented. The comments are limited to the essentials. All cultivable species present in the inoculum were detected in the biofilm microbiota of the controls immediately after the saline rinse and 24h later. All species increased during this time period. The colonization density of *P*. *gingivalis* was, however low. *T*. *denticola* could be detected in addition microscopically.

TI brushing alone followed by a saline rinse had a clear immediate reducing effect on the number of all species and also the numbers of all species increased during the following 24 h. TI brushing followed by 0.2% CHX exposure had a modest immediate effect. After 24 h, the characteristic sustained action of CHX was evident for all species, albeit in a species specific manner. Most susceptible were the Gram negative anaerobes.

TI brushing followed by an exposure to 1 weight% NaClO solution had a drastic immediate effect on all species eradicating the CFU below detection limit. Also characteristic was the pronounced survival of all species with the exception of *P..intermedia* and *P. gingivalis* after 24h as well as the pronounced variation among the surviving species.

Reduction of the NaClO solution concentration to 0.1 weight% after using Ti- brush showed similar results as with the 1.0 weight% solution. It is most probable the lack of a concentration effect is due to the fact that these concentrations were tested in 2 separate biofilm experiments.

As already mentioned, after using Ti- brush followed with exposures to 1 weight% NaClO solution and 0.2 weight% CHX solution in sequence led to a superb result eradicating all bacteria immediately and also after 24h. (see figure 6).

It might be added that the eradication of *P. intermedia* and *P.gingivalis* might be explained by the fact that these species colonized these biofilms in lower numbers in comparison to the other species. This must also be kept in mind when viewing the results of all treatments tested in these experiments.

### Experiment 5

### Evaluation of subgingival biofilm decontamination with various antimicrobial solutions tested in a subgingival biofilm model

For a detailed description of the biofilm model used in this experiment, see e.g. Guggenheim et al., 2004, 2001a; and Shapiro et al., 2002 and Guggenheim et al., 2009.

### STRAINS:

OMZ 278, *Prevotella intermedia*
OMZ 493, *Veilonella dispar*
OMZ 598, *Fusobacterium nucleatum*
OMZ 607, *Streptococcus oralis*
OMZ 661, *Treponema denticola*
OMZ 698, *Campylobacter rectus*
OMZ 745, *Actinomyces oris*
OMZ 871, *Streptococcus anginosus*
OMZ 925, *Porphyromonas gingivalis*
OMZ 1047, *Tannerella forsythia*

### TEST SOLUTIONS:

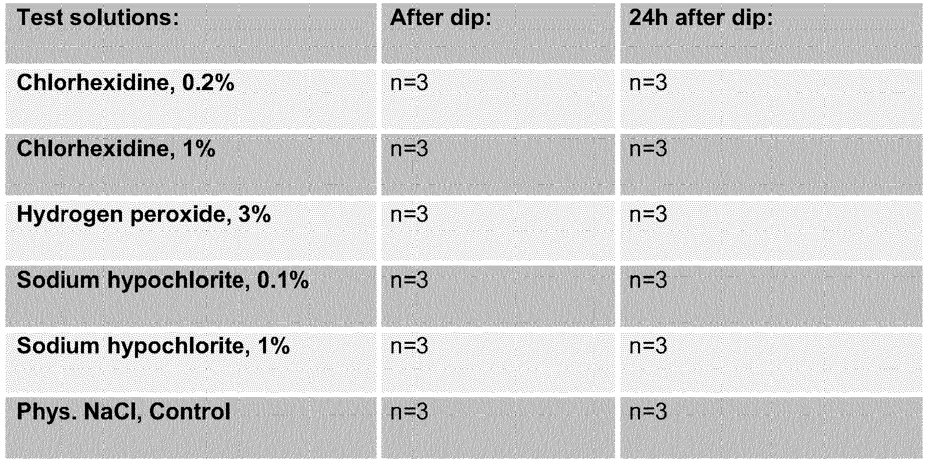

### RESULTS:

The 2 biofilm experiments presented here were a continuation of the biofilm trials reported under experiment 4, including in part minor changes in the treatment sequence, new antimicrobials and changes in concentration. Again, it included several treatments and had to be split into two parts because of the demanding requirements of the test design in particular with regard to timing. To be able to compare the results of the 2 experiments, the NaCl control had to be repeated in both experiments.

An overview of the results of both experiments is provided in figure 16. As in experiment 4, the saline controls (0 and 24 h) fluctuated within 1 log step allowing a direct comparison of all treatments (data 2.0). The titanium discs were colonized immediately after rinsing with saline by 2.1E8 CFU and slightly increased after 24 h to 3.9 E8 CFU. The combination of TiBrush® and 0.2 weight% CHX solution, reduced microbial colonization immediately after treatment by = 4 log steps but regrowth after 24 h was evident similar to experiment 4 (see figure 5).

Using TiBrush® + NaClO solution 0.1% had a very good immediate effect but regrowth after 24 h was again pronounced (see figure 17).

Again, an impressing result was achieved by TI brushing and a combination of rinsing with 0.1 weight% NaClO solution followed by a rise with 0.2 weight% CHX solution. Here the biofilm microbiota was reduced immediately after treatment and also 24h later below detection limit (see figure 18).

### SUMMARY:

All cultivable species present in the inoculum and were detected in the biofilm microbiota of the controls immediately as well as after the saline rinse and 24h later. The colonization density of *P*. *gingivalis* was, however low. *T*. *denticola* could be detected in addition microscopically.

The Gram-negative anaerobes (*P. intermedia, P. gingivalis* and *Fusobacterium nucleatum*) were very susceptible to all antimicrobials used in this experiment. *Tannerella forsythia* and *Treponema denticola* could not be assessed by culture techniques. All the other facultative anaerobes showed an increased resistance against the antimicrobials used in this experiment with the exception of NaClO solution+CHX solution.

### Experiment 6

### Evaluation of subgingival biofilm regrowth after decontamination with various antimicrobial solutions tested in a subgingival biofilm model

For a detailed description of the biofilm model used in this experiment, see e.g. Guggenheim et al., 2004, 2001a; and Shapiro et al., 2002 and Guggenheim et al., 2009.

### STRAINS:

OMZ 278, *Prevotella intermedia*
OMZ 493, *Veilonella dispar*
OMZ 598, *Fusobacterium nucleatum*
OMZ 607, *Streptococcus oralis*
OMZ 661, *Treponema denticola*
OMZ 698, *Campylobacter rectus*
OMZ 745, *Actinomyces oris*
OMZ 871, *Streptococcus anginosus*
OMZ 925, *Porphyromonas gingivalis*
OMZ 1047, *Tannerella forsythia*

### TREATMENT:

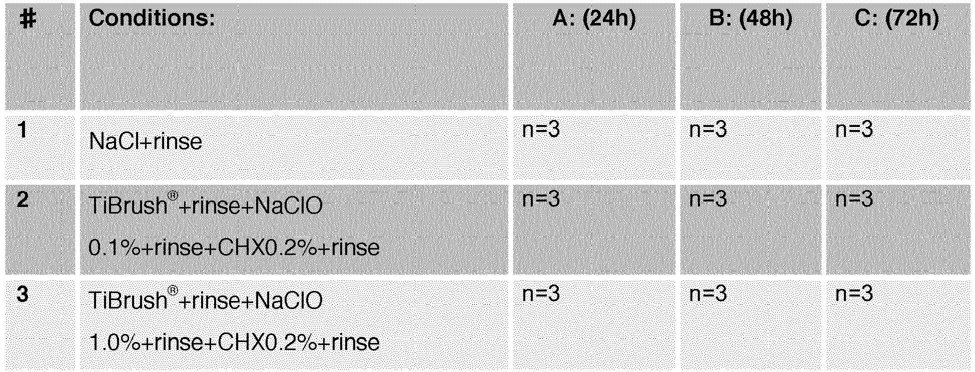

### RESULTS:

The results of this biofilm study are most impressive. Even 72 h after treatment, the reduction of the biofilm microbiota (total CFU) was still highly significant when compared to the respective saline control. Rather unexpected and hard to explain are the findings of the 72 h regrowth where the 0.1 weight% NaClO solution values showed to be lower than the 0.2 weight% NaClO solution containing treatment (figures 20 and 21).

Most importantly, the present findings disclose the selective effects of these treatments on single species (figures 19, 20 and 21). The putative pathogenic, Gram-negative anaerobic bacteria strongly associated with inflammation and peri-implantitis (*F. nucleatum, P. intermedia. and P. gingivalis*) were after the 72 h regrowth period still below detection level. *Campylobacter rectus,* a micro-aerophilic species also less strongly associated with inflammation was completely suppressed after 48 h and significantly reduced in the biofilms after 72 h. The Gram-positive species, as well as *V. dispar,* not associated with periodontitis and peri-implantitis, were able to regrow only after 72 h in a treatment dependent manner. These species are only weakly associated with inflammation or even regarded as part of a microbiota associated with health.

### Experiment 7

The aim of the study was to evaluate the efficiency and effectiveness of several new treatment protocols combining mechanical debridement (TiBrush®) and decontamination with cleaning agents (CHX, NaClO) on human biofilms. The study consisted of three activities described below.

Comparison of data on Clean Implant Surface (CIS) after treatment of Straumann SLA® discs with TiBrush® and cleaning agents in a human in-vivo splint model, is shown in figure 6.

### MATERIAL AND METHODS:

### Study samples and study groups

Intraoral splints are used to collect an in vivo supragingival plaque biofilm on Ø 15 mm titanium sand-blasted large grit and acid etched (SLA®) discs after 48 h. Per test persons and test run 4 discs of the same type are applied with the intraoral splint. The discs are equally and randomly assigned to the subsequent treatment methods after collection from patients.

### Study population

Healthy volunteers are included in the study. The criteria needed for inclusion are: (1) no systemic use of antibiotics during the last 6 months, (2) good level of oral hygiene (PI <25% BI <25o/o), (3) no signs of destructive periodontitis or any inflammatory conditions of the surrounding soft tissues, and (4) nonsmoker. Prior to the investigation, the subjects receive a professional tooth cleaning. The volunteers obtain open acrylic appliances for the upper jaw with four discs (Ø15 mm, 1 mm thick) to collect a supragingival plaque biofilm. Specimens are inserted into depressions with sticky wax towards the palate in a distance of-1-2 mm to provide a nutritious aqueous environment. The splints are worn by the volunteers for the defined durations. The subjects are allowed to maintain their regular diet and retain the splints intra-orally throughout the whole experimental period, except during eating and during their daily mechanical tooth-brushing only with advised toothpaste (containing Natriumfluorid) twice daily (morning and evening) and subsequent thorough rinsing with tab water (no mouth-rinses are used). During removal from the oral cavity, for purpose of eating and tooth cleaning, the splints are stored in water.

### Sequential cleaning procedure

The discs used are 15mm discs. Experiments are carried out at room temperature. The time between the removal of the discs from the subjects until treatment start, as well as the time between the last step in the treatment procedure and the following analyses of the biofilm is standardized and kept as short as possible.

### Histomorphometrical analysis

After biofilm formation all samples were treated in the same way. Immediately after plaque collection period (48 hours), splints were removed from the oral cavity and specimens were extracted from the splint, gently rinsed with water and 40 samples were stained with erythrosine (Erythrosine B, Certistain®, Merck KGaA, Darmstadt, Germany). These samples were photographed at a magnification of 8 by the use of a stereo microscope (SZ61, Olympus Europa Holding GmbH, Hamburg, Germany) and a digital camera (ColorViewlll, Olympus Holding GmbH, Hamburg, Germany). For analysing the surfaces of samples a professional image and documentation software (Cell D, Olympus Europa GmbH, Hamburg, Germany) was used. Ten measurements were taken per sample by random placing square fields on the sample surface. The initial plaque surface (IPS) was determined in this way. After performance of the different cleaning procedures a second histomorphometrical analysis was done according to the above mentioned steps for evaluation of the residual plaque areas (RPA).

The use of TiBrush® + NaClO + CHX, at concentrations indicated below, resulted in a significant higher biofilm removal than obtained by using TiBrush® alone or TiBrush® + 0.2% CHX. Both the sequential treatments TiBrush® + 0.1% NaOCl + 0.2% CHX and TiBrush® + 1% NaOCl + 0.2% CHX as well as the treatment with combined application of TiBrush® and 0.1% NaOCl followed by 0.2% CHX showed a nearly complete clean surface, as seen in Figure 6.

### List of References

1. Socransky and Haffajee, 2000,
2. Socransky and Haffajee, 2000
3. Socransky et al., Periodontology 2000, Vol. 28, 2002, 12-55*.*
4. Guggenheim et al, 2004,
5. Guggenheim et al, 2001a;
6. Guggenheim et al, BMC Microbiology 2009, 9:280 doi:10.1186/1471-2180-9-280.
7. Shapiro et al., 2002
8. WO 2009/083281
9. WO 2011/152789
10. Schwartz et al., 2006

## Claims

1. A kit of parts for use in treating a periodontal disease, selected from the group consisting of periimplantitis, gingivitis, periodontitis and periimplant mucositis, comprising:
a) a 0.01 to 1 weight% sodium hypochlorite (NaClO) solution;
b) a 0.01 to 1 weight%, chlorhexidine (CHX) solution,
wherein the solutions are sequentially administered to a site of microbial infection in a patient so that the sodium hypochlorite (NaClO) solution is administered before the chlorhexidine (CHX) solution.

2. A kit of parts for use according to claim 1, wherein the NaClO solution has a concentration of 0.1 weight% and the CHX solution has a concentration of 0.2 weight%.

3. A kit of parts for use according to claims 1 or 2, wherein said kit of parts further comprises a mechanical cleaning and/or debridement tool to be used before and/or together with the sodium hypochlorite (NaClO) solution.

4. A kit of parts for use according to claim 3, wherein said mechanical cleaning and/or debridement tool is selected from the group consisting of a cuvette, drill, brush, ultrasonic device and spatula.

5. A kit of parts for use according to claim 4, wherein said mechanical cleaning and/or debridement tool is a titanium bristled brush.

6. A kit of parts for use according to any one of claims 1-5, wherein said kit of parts comprises at least one further cleaning and/or disinfecting agent, which is to be administered before, after and/or together with the sodium hypochlorite (NaClO) solution and/or the chlorhexidine (CHX) solution.

7. A kit of parts for use according to any one of claims 1-6, wherein said kit of parts comprises at least one further rinsing solution agent, which is to be administered between the sodium hypochlorite (NaClO) solution and the chlorhexidine (CHX) solution.

8. A kit of parts for use according to any one of claims 1-7, wherein the instructions are written instructions.

9. A kit of parts comprising at least two cleaning and/or disinfecting agents for use in treating a periodontal disease, selected from the group consisting of periimplantitis, gingivitis, periodontitis and periimplant mucositis, wherein two of said cleaning and/or disinfecting agents are selected from
a) a 0.01 to 1 weight% sodium hypochlorite (NaClO) solution and
b) a 0.01 to 1 weight%, chlorhexidine (CHX) solution,
wherein the solutions are sequentially administered to a site of microbial infection in a patient so that the sodium hypochlorite (NaClO) solution is administered before the chlorhexidine (CHX) solution.

10. A kit of parts comprising at least two cleaning and/or disinfecting agents for use according to claim 9, wherein said kit further comprises a mechanical cleaning and/or debridement tool to be used before and/or together with the sodium hypochlorite (NaClO) solution and/or the chlorhexidine (CHX) solution.

11. A kit of parts comprising at least two cleaning and/or disinfecting agents for use according to claim 10, wherein said mechanical cleaning and/or debridement tool is selected from the group consisting of a cuvette, drill, brush, ultrasonic device and spatula.

12. A kit of parts comprising at least two cleaning and/or disinfecting agents for use according to claim 10, wherein said mechanical cleaning and/or debridement tool is a titanium bristled brush.

13. A kit of parts comprising at least two cleaning and/or disinfecting agents for use according to any one of claims 9-12, wherein said kit of parts comprises one or more rinsing solutions to be applied between the sodium hypochlorite (NaClO) solution and the chlorhexidine (CHX) solution.

14. A kit of parts comprising at least two cleaning and/or disinfecting agents for use according to any one of claims 9-13, wherein the NaClO solution has a concentration of 0.1 weight%, and the CHX solution has a concentration of 0.2 weight%.

15. A sequential release formulation for treating a periodontal disease, selected from the group consisting of periimplantitis, gingivitis, periodontitis and periimplant mucositis, in a patient suffering from a periodontal disease, comprising at least two cleaning agents selected from:
a) a 0.01 to 1 weight% sodium hypochlorite (NaClO) solution and
b) a 0.01 to 1 weight%, chlorhexidine (CHX) solution,
wherein said agents are released in a given sequential order of first a) and thereafter b).

16. A formulation according to claim 15, wherein the NaClO solution has a concentration of 0.1 weight%, and the CHX solution has a concentration of 0.2 weight%.

## Patentansprüche

1. Teilesatz zur Verwendung bei der Behandlung einer Parodontalerkrankung, ausgewählt aus der Gruppe bestehend aus Periimplantitis, Gingivitis, Periodontitis und periimplantärer Mukositis, umfassend:
a) eine Lösung mit 0,01 bis 1 Gew.-% Natriumhypochlorit (NaClO) ;
b) eine Lösung mit 0,01 bis 1 Gew.-% Chlorhexidin (CHX),
wobei die Lösungen sequenziell an einen Ort einer mikrobiellen Infektion bei einem Patienten verabreicht werden, so dass die Natriumhypochloritlösung (NaClO-Lösung) vor der Chlorhexidin-Lösung (CHX-Lösung) verabreicht wird.

2. Teilesatz zur Verwendung nach Anspruch 1, wobei die NaClO-Lösung eine Konzentration von 0,1 Gew.-% aufweist und die CHX-Lösung eine Konzentration von 0,2 Gew.-% aufweist.

3. Teilesatz zur Verwendung nach Anspruch 1 oder 2, wobei der Teilesatz ferner ein mechanisches Reinigungs- und/oder Debridementwerkzeug umfasst, welches vor und/oder zusammen mit der Natriumhypochloritlösung (NaClO-Lösung) verwendet wird.

4. Teilesatz zur Verwendung nach Anspruch 3, wobei das mechanische Reinigungs- und/oder Debridementwerkzeug ausgewählt ist aus der Gruppe bestehend aus einer Küvette, einem Bohrer, einer Bürste, einer Ultraschallvorrichtung und einem Spatel.

5. Teilesatz zur Verwendung nach Anspruch 4, wobei das mechanische Reinigungs- und/oder Debridementwerkzeug eine Titanborstenbürste ist.

6. Teilesatz zur Verwendung nach einem der Ansprüche 1-5, wobei der Teilesatz mindestens ein weiteres Reinigungs- und/oder Desinfektionsmittel umfasst, welches vor, nach und/oder zusammen mit der Natriumhypochloritlösung (NaClO-Lösung) und/oder der Chlorhexidinlösung (CHX-Lösung) verabreicht wird.

7. Teilesatz zur Verwendung nach einem der Ansprüche 1-6, wobei der Teilesatz mindestens ein weiteres Spüllösungsmittel umfasst, welches zwischen der Natriumhypochloritlösung (NaClO-Lösung) und der Chlorhexidinlösung (CHX-Lösung) verabreicht wird.

8. Teilesatz zur Verwendung nach einem der Ansprüche 1-7, wobei die Anweisungen schriftliche Anweisungen sind.

9. Teilesatz umfassend mindestens zwei Reinigungs- und/oder Desinfektionsmittel zur Verwendung bei der Behandlung einer Parodontalerkrankung, ausgewählt aus der Gruppe bestehend aus Periimplantitis, Gingivitis, Periodontitis und periimplantärer Mukositis, wobei zwei der Reinigungs- und/oder Desinfektionsmittel ausgewählt sind aus
a) einer Lösung mit 0,01 bis 1 Gew.-% Natriumhypochlorit (NaClO) und
b) einer Lösung mit 0,01 bis 1 Gew.-% Chlorhexidin (CHX),
wobei die Lösungen sequenziell an einen Ort einer mikrobiellen Infektion bei einem Patienten verabreicht wird, so dass die Natriumhypochloritlösung (NaClO-Lösung) vor der Chlorhexidinlösung (CHX-Lösung) verabreicht wird.

10. Teilesatz umfassend mindestens zwei Reinigungs- und/oder Desinfektionsmittel zur Verwendung nach Anspruch 9, wobei der Teilesatz ferner ein mechanisches Reinigungs- und/oder Debridementwerkzeug umfasst, welches vor und/oder zusammen mit der Natriumhypochloritlösung (NaClO-Lösung) und/oder der Chlorhexidinlösung (CHX-Lösung) verwendet wird.

11. Teilesatz umfassend mindestens zwei Reinigungs- und/oder Desinfektionsmittel zur Verwendung nach Anspruch 10, wobei das mechanische Reinigungs- und/oder Debridementwerkzeug ausgewählt ist aus der Gruppe bestehend aus einer Küvette, einem Bohrer, einer Bürste, einer Ultraschallvorrichtung und einem Spatel.

12. Teilesatz umfassend mindestens zwei Reinigungs- und/oder Desinfektionsmittel zur Verwendung nach Anspruch 10, wobei das mechanische Reinigungs- und/oder Debridementwerkzeug eine Titanborstenbürste ist.

13. Teilesatz umfassend mindestens zwei Reinigungs- und/oder Desinfektionsmittel zur Verwendung nach einem der Ansprüche 9-12, wobei der Teilesatz mindestens eine oder mehrere Spüllösungen umfasst, welche zwischen der Natriumhypochloritlösung (NaClO-Lösung) und/oder der Chlorhexidinlösung (CHX-Lösung) verabreicht wird.

14. Teilesatz umfassend mindestens zwei Reinigungs- und/oder Desinfektionsmittel zur Verwendung nach einem der Ansprüche 9-13, wobei die NaClO-Lösung eine Konzentration von 0,1 Gew.-% aufweist und die CHX-Lösung eine Konzentration von 0,2 Gew.-% aufweist.

15. Formulierung zur sequenziellen Freisetzung zur Behandlung einer Parodontalerkrankung, ausgewählt aus der Gruppe bestehend aus Periimplantitis, Gingivitis, Periodontitis und periimplantärer Mukositis bei einem Patienten, welcher an einer Parodontalerkrankung leidet, umfassend mindestens zwei Reinigungsmittel ausgewählt aus:
a) einer Lösung mit 0,01 bis 1 Gew.-% Natriumhypochlorit (NaClO) und
b) einer Lösung mit 0,01 bis 1 Gew.-% Chlorhexidin (CHX),
wobei die Mittel in einer gegebenen sequenziellen Reihenfolge von erst a) und danach b) freigesetzt werden.

16. Formulierung nach Anspruch 15, wobei die NaClO-Lösung eine Konzentration von 0,1 Gew.-% aufweist und die CHX-Lösung eine Konzentration von 0,2 Gew.-% aufweist.

## Revendications

1. Kit de pièces pour utilisation dans le traitement d'une maladie parodontale, choisie dans le groupe constitué de la péri-implantite, de la gingivite, de la parodontite et de la mucosité péri-implantaire, comprenant :
a) une solution d'hypochlorite de sodium (NaClO) de 0,01 à 1 % en poids ;
b) une solution de chlorhexidine (CHX) de 0,01 à 1 % en poids,
dans lequel les solutions sont administrées séquentiellement à un site d'infection microbienne chez un patient de sorte que la solution d'hypochlorite de sodium (NaClO) est administrée avant la solution de chlorhexidine (CHX) .

2. Kit de pièces pour utilisation selon la revendication 1, dans lequel la solution de NaClO a une concentration de 0,1 % en poids et la solution de CHX a une concentration de 0,2 % en poids.

3. Kit de pièces pour utilisation selon les revendications 1 ou 2, dans lequel ledit kit de pièces comprend en outre un outil de nettoyage et/ou de débridement mécanique à utiliser avant et/ou avec la solution d'hypochlorite de sodium (NaClO).

4. Kit de pièces pour utilisation selon la revendication 3, dans lequel ledit outil de nettoyage et/ou de débridement mécanique est choisi dans le groupe constitué d'une cuvette, d'une perceuse, d'une brosse, d'un dispositif à ultrasons et d'une spatule.

5. Kit de pièces pour utilisation selon la revendication 4, dans lequel ledit outil de nettoyage et/ou de débridement mécanique est une brosse en poils de titane.

6. Kit de pièces pour utilisation selon l'une quelconque des revendications 1 à 5, dans lequel ledit kit de pièces comprend au moins un autre agent de nettoyage et/ou de désinfection, qui doit être administré avant, après et/ou avec la solution d'hypochlorite de sodium (NaClO) et/ou la solution de chlorhexidine (CHX).

7. Kit de pièces pour utilisation selon l'une quelconque des revendications 1 à 6, dans lequel ledit kit de pièces comprend au moins un autre agent de solution de rinçage, qui doit être administré entre la solution d'hypochlorite de sodium (NaClO) et la solution de chlorhexidine (CHX).

8. Kit de pièces pour utilisation selon l'une quelconque des revendications 1 à 7, dans lequel les instructions sont des instructions écrites.

9. Kit de pièces comprenant au moins deux agents de nettoyage et/ou de désinfection pour utilisation dans le traitement d'une maladie parodontale, choisie dans le groupe constitué de la péri-implantite, de la gingivite, de la parodontite et de la mucosité péri-implantaire, dans lequel deux desdits agents nettoyants et/ou désinfectants sont choisis parmi
a) une solution d'hypochlorite de sodium (NaClO) de 0,01 à 1 % en poids et
b) une solution de chlorhexidine (CHX) de 0,01 à 1 % en poids,
dans lequel les solutions sont administrées séquentiellement à un site d'infection microbienne chez un patient de sorte que la solution d'hypochlorite de sodium (NaClO) est administrée avant la solution de chlorhexidine (CHX) .

10. Kit de pièces comprenant au moins deux agents de nettoyage et/ou de désinfection pour utilisation selon la revendication 9, dans lequel ledit kit comprend en outre un outil de nettoyage et/ou de débridement mécanique à utiliser avant et/ou avec la solution d'hypochlorite de sodium (NaClO) et/ou la solution de chlorhexidine (CHX).

11. Kit de pièces comprenant au moins deux agents de nettoyage et/ou de désinfection pour utilisation selon la revendication 10, dans lequel ledit outil de nettoyage et/ou de débridement mécanique est choisi dans le groupe constitué d'une cuvette, d'une perceuse, d'une brosse, d'un dispositif à ultrasons et d'une spatule.

12. Kit de pièces comprenant au moins deux agents de nettoyage et/ou de désinfection pour utilisation selon la revendication 10, dans lequel ledit outil de nettoyage et/ou de débridement mécanique est une brosse en poils de titane.

13. Kit de pièces comprenant au moins deux agents de nettoyage et/ou de désinfection pour utilisation selon l'une quelconque des revendications 9 à 12, dans lequel ledit kit de pièces comprend une ou plusieurs solutions de rinçage à appliquer entre la solution d'hypochlorite de sodium (NaClO) et la solution de chlorhexidine (CHX).

14. Kit de pièces comprenant au moins deux agents de nettoyage et/ou de désinfection pour utilisation selon l'une quelconque des revendications 9 à 13, dans lequel la solution de NaClO a une concentration de 0,1 % en poids et la solution de CHX a une concentration de 0,2 % en poids.

15. Formulation à libération séquentielle pour le traitement d'une maladie parodontale, choisie dans le groupe constitué de la péri-implantite, de la gingivite, de la parodontite et de la mucosité péri-implantaire, chez un patient souffrant d'une maladie parodontale, comprenant au moins deux agents de nettoyage choisis parmi :
a) une solution d'hypochlorite de sodium (NaClO) de 0,01 à 1 % en poids et
b) une solution de chlorhexidine (CHX) de 0,01 à 1 % en poids,
dans lequel lesdits agents sont libérés dans un ordre séquentiel donné du premier a) et ensuite de b).

16. Formulation selon la revendication 15, dans laquelle la solution de NaClO a une concentration de 0,1 % en poids et la solution de CHX a une concentration de 0,2 % en poids.
